# FASCICULE DE BREVET EUROPEEN

(11) **EP 3 302 803 B1**
(45) Date de publication et mention de la délivrance du brevet: **11.12.2019**
(21) Numéro de dépôt: 16739203.4
(22) Date de dépôt: 06.06.2016
(51) Int. Cl.: B01L 3/00

(54) **PROCEDE ET DISPOSITIF POUR HYDRATER PAR UN ECHANTILLON LIQUIDE UN SUPPORT HYDRATANT**
VERFAHREN UND VORRICHTUNG ZUR HYDRATISIERUNG EINES HYDRATISIERUNGSMEDIUMS DURCH EINE FLÜSSIGE PROBE
METHOD AND DEVICE FOR HYDRATING A HYDRATING MEDIUM BY A LIQUID SAMPLE

(30) Priorité: 05.06.2015 FR 1555145
(43) Date de publication de la demande: 11.04.2018
(73) Titulaire: Biomérieux, 69280 Marcy-L'Etoile (FR)
(72) Inventeur: COLIN, Bruno, 69280 Marcy L'Etoile (FR); DÉVIGNE, Laurence, 01150 Vaux En Bugey (FR); GHIRARDI, Sandrine, 69290 Saint Genis Les Ollières (FR); MONTET, Marie-Pierre, 69200 Vénissieux (FR); ROZAND, Christine, 69290 Saint Genis Les Ollières (FR); WANDELS, Philippe, 69003 Lyon (FR)
(74) Mandataire: Cabinet Beau de Loménie
(86) Numéro de dépôt international: PCT/FR2016/051346
(87) Numéro de publication internationale: WO 2016/193647

(56) Documents cités:
- EP-A1- 1 415 788
- EP-A2- 1 174 716
- WO-A2-2005/012549

## Description

La présente invention concerne le domaine technique de l'hydratation par un échantillon liquide, d'un support hydratant comprenant un milieu réactionnel au sens général.

L'objet de l'invention trouve des applications avantageuses dans de nombreux domaines dans lesquels il apparaît le besoin d'activer par un liquide, un milieu réactionnel préalablement intégré à un support hydratant.

Par exemple, l'objet de l'invention trouve une application particulièrement avantageuse dans le domaine des supports hydratants imprégnés d'un milieu réactionnel et qui nécessitent juste avant utilisation, une réhydratation pour activer le milieu réactionnel et les rendre prêts à l'emploi.

Une application particulièrement préférée se trouve dans le contrôle microbiologique. Il requiert la mise en oeuvre de techniques qui permettent la détection - par exemple aux fins de détection et/ou de dénombrement - de micro-organismes et dont le rendu en termes de résultats doit être le plus rapide possible. D'une manière générale, lesdits micro-organismes peuvent être non pathogènes, telles les bactéries d'intérêts technologiques comme les ferments ou les indicateurs de qualité ; ces derniers permettant de valider le processus de production, de matières premières aux produits bruts, jusqu'aux produits finis. Toutefois, les micro-organismes faisant l'objet d'un contrôle microbiologique sont le plus fréquemment pathogènes, nécessitant dès lors une détection rapide et précise de ceux-ci afin d'engager les actions correctives ad hoc le plus tôt possible. Bien évidemment, les toxines produites par de tels micro-organismes pathogènes - et responsables en tout ou partie de leur caractère pathogène - peuvent également être recherchées.

Dans tous les cas, et quelle que soit l'origine de l'échantillon faisant l'objet d'un contrôle microbiologique, les facteurs importants pour évaluer l'efficacité de ce contrôle sont : la sensibilité, la spécificité et le temps d'obtention du résultat (« time-to-result », en langue anglaise).

Ainsi une application de l'invention particulièrement avantageuse est de détecter et/ou identifier et/ou énumérer, au moins un organisme dans un échantillon liquide susceptible de le contenir.

Une application préférée de l'objet de l'invention vise à déterminer la sensibilité et la résistance à au moins un antibiotique, d'au moins un microorganisme susceptible d'être contenu dans un échantillon liquide.

L'art antérieur a proposé de nombreuses solutions techniques pour identifier ou détecter un analyte ou un organisme vivant dans un échantillon liquide.

Ainsi, le brevet EP 0 319 294 décrit une méthode et un dispositif permettant de déterminer la présence d'un analyte dans un échantillon liquide. Ce dispositif comprend d'une part, une cellule de réaction comprenant une membrane microporeuse portée par un matériau absorbant et d'autre part, un applicateur de l'échantillon liquide se présentant sous la forme d'un tube ayant un orifice avec une surface ouverte sensiblement plus petite que la surface de la membrane. Ce dispositif comporte également un système réactif comprenant des composants capables de coopérer avec l'analyte immobilisé dans la membrane microporeuse pour produire un signal visuel. L'applicateur est mis en contact, par son orifice, avec la membrane pour concentrer l'analyte de l'échantillon et/ou les composants du réactif dans la surface de contact pour ainsi produire un signal visuel ayant les dimensions de l'orifice. Si un tel dispositif permet de concentrer la réaction pour une meilleure visualisation, un tel dispositif s'avère de mise en œuvre délicate en ce qui concerne notamment le contrôle du transfert de l'échantillon liquide sur la membrane et la mise en place du système réactif pour obtenir la réaction avec l'analyte de l'échantillon.

Le brevet US 8 343 726 décrit un dispositif de détection de substances présentes dans un liquide. Ce dispositif comporte d'une part, un récipient contenant un matériau poreux de réception d'un échantillon liquide et d'autre part, une membrane poreuse imprégnée d'un milieu de culture. La membrane poreuse est destinée à être placée en position de superposition par rapport au récipient pour venir en contact avec l'échantillon liquide qui diffuse dans la membrane. Un tel dispositif présente l'inconvénient de ne pas pouvoir contrôler l'hydratation de la membrane poreuse par l'échantillon liquide. Or, la migration de l'échantillon liquide à l'intérieur du support hydratant doit être maîtrisé afin de ne pas perturber la répartition du milieu réactionnel dans le support hydratant et par suite, modifier les conditions de réaction. Inversement, l'échantillon liquide doit être capable d'hydrater la totalité du support hydratant afin que ce dernier offre sa capacité optimale de réaction.

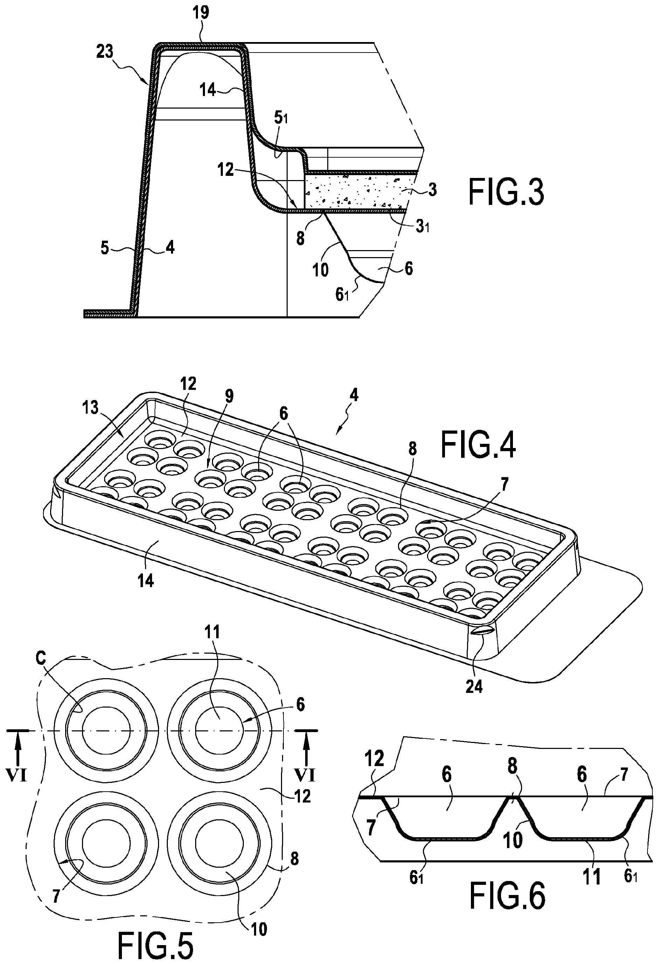

Dans le même sens, la demande de brevet EP 1 415 788 décrit un dispositif microfluidique pour l'analyse chimique et biologique comportant dans l'exemple de réalisation à la Figure 5, d'une part un composant microfluidique présentant des puits et d'autre part un couvercle pourvu de substrats portant une matrice de réactifs. Lorsque le couvercle est placé au-dessus du composant microfluidique, le substrat se trouve placé dans la chambre définie par le puits pour être hydraté par le milieu liquide contenu dans le composant microfluidique.

Cet exemple de réalisation du dispositif microfluidique ne donne aucune indication sur la capacité à contrôler l'hydratation du substrat par le milieu liquide. Cependant, dans l'exemple de réalisation illustré à la Figure 4, le substrat portant la matrice de réactifs est positionné complètement à l'intérieur du puits de sorte que l'hydratation du substrat par le milieu liquide n'est pas contrôlée.

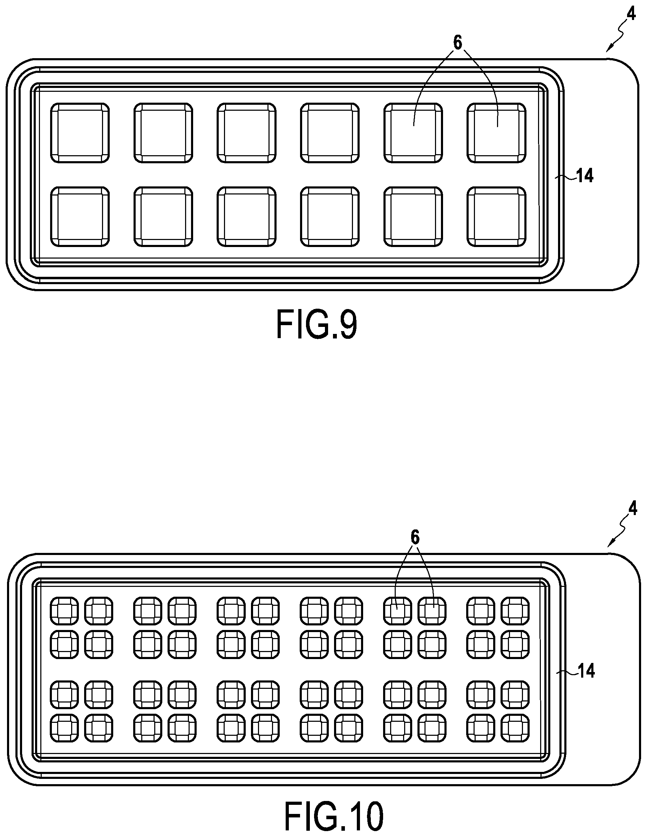

Dans le domaine technique des biocapteurs, la demande de brevet EP 1 174 716 décrit un biocapteur comportant un substrat muni d'électrodes et présentant un puits de réception d'un échantillon liquide, pourvu d'un réactif de test. Ce biocapteur est pourvu d'un couvercle permettant de protéger la zone test avant et après utilisation. Selon la variante de réalisation illustrée aux Figures 9 à 11, le couvercle est pourvu d'un substrat d'absorption du liquide excédentaire. Ce dispositif qui propose de verser le milieu liquide dans le puits ne permet pas de contrôler l'hydratation du substrat par ce milieu liquide.

Dans le domaine technique des membranes, la demande de brevet WO 2005/012549 décrit un appareil pour évaluer les propriétés de barrière d'une membrane. Cet appareil comporte une membrane test montée entre une plaque de donneur et une plaque de réception. La plaque de donneur est aménagée pour comporter des puits d'échantillons de liquide en relation desquels des électrodes sont montées. Cet appareil qui vise à caractériser les caractéristiques des membranes ne contrôle pas l'hydratation de la membrane par l'échantillon liquide.

L'objet de l'invention vise à remédier aux inconvénients de l'art antérieur en proposant une nouvelle technique permettant de contrôler, de manière simple et sûre, l'hydratation d'un support hydratant par un échantillon liquide, sans générer de perturbations dans le milieu réactionnel du support hydratant tout en optimisant les conditions de réaction du milieu réactionnel avec l'échantillon liquide.

Pour atteindre cet objectif, l'objet de l'invention concerne un dispositif pour hydrater par un échantillon liquide, un support hydratant comprenant un milieu réactionnel comprenant tous les éléments nécessaires à la survie et/ou à la croissance des microorganismes, le dispositif comportant :
- un réceptacle de réception d'au moins un échantillon liquide, ce réceptacle comprenant au moins un puits de réception ouvert vers le haut au niveau d'un bord supérieur ;
- un couvercle possédant une face inférieure sur laquelle au moins un support hydratant est fixé pour présenter une face d'absorption pour un échantillon liquide lorsque le couvercle et le réceptacle occupent une position d'hydratation dans laquelle le support hydratant est en contact avec l'échantillon liquide selon une interface d'hydratation.

Selon l'invention, le réceptacle présente, par au moins un puits, un volume calibré de réception d'un échantillon liquide, l'au moins dit puits présentant une section ouverte calibrée d'hydratation d'un support hydratant délimitée par le bord supérieur d'au moins ledit puits, cette section ouverte calibrée d'hydratation possédant une surface inférieure à la surface de la face d'absorption du support hydratant pour assurer le contrôle de l'absorption par capillarité, de l'échantillon liquide par le support hydratant.

Le dispositif selon l'invention permet d'une part, de doser simplement et avec précision, le volume de l'échantillon liquide nécessaire pour réagir avec la totalité du milieu réactionnel du support hydratant et d'autre part, de maîtriser les conditions de mise en contact de l'échantillon liquide avec le milieu réactionnel afin d'optimiser les conditions d'activation et/ou de réaction.

Selon une caractéristique avantageuse de réalisation, la surface de la section ouverte calibrée d'hydratation du support hydratant par un échantillon liquide délimitée par au moins un puits est telle que le ratio de cette surface de la section calibrée pour l'hydratation d'un support hydratant par un échantillon liquide, sur la surface de la face d'absorption du support hydratant est compris entre 40 et 80 % et de préférence entre 50 et 75 % et avantageusement entre 55 et 65 %.

Selon une variante préférée de réalisation, le réceptacle comporte des puits de réception pour plusieurs échantillons liquides et le couvercle est équipé de plusieurs supports hydratants espacés les uns des autres par des couloirs de séparation et destinés, en position d'hydratation, à être hydratés chacun par un échantillon liquide.

Cette variante de réalisation permet d'hydrater plusieurs supports hydratants tout en évitant les contaminations croisées entre les supports hydratants et par suite, les échantillons liquides. Les couloirs de séparation permettent également de faire circuler l'air entre les supports hydratants pour favoriser la croissance de certaines bactéries durant l'incubation.

Selon un mode de réalisation, la surface de la section ouverte calibrée d'hydratation d'un support hydratant par un échantillon liquide est délimitée par plusieurs puits présentant chacun une section ouverte calibrée d'hydratation et qui, en combinaison avec les autres sections des puits, présentent au total, une surface correspondant à la surface de la section ouverte calibrée d'hydratation pour un support hydratant. Ce mode de réalisation permet de répartir le front de contact entre l'échantillon liquide et la surface du support hydratant, en choisissant le nombre de puits en fonction de la surface du support hydratant. Par ailleurs, la répartition de l'échantillon liquide dans plusieurs puits optimise le dosage de l'échantillon liquide.

Selon un autre mode avantageux de réalisation, chaque puits comporte plusieurs cavités de réception pour une partie de l'échantillon liquide, ces cavités de réception communiquant entre-elles au moins au niveau de la section ouverte calibrée d'hydratation dont le contour possède différents lobes. La répartition de l'échantillon liquide dans diverses cavités permet un meilleur dosage du liquide à analyser, ce qui est important si celui-ci est légèrement visqueux (cas du lait par exemple ou des échantillons liquides dilués).

Selon un autre mode de réalisation, la surface de la section ouverte calibrée du réceptacle pour un échantillon liquide est délimitée par un unique puits. Ce mode de réalisation permet d'améliorer la visibilité du test au travers le dispositif lors de l'étape de lecture.

Selon une caractéristique avantageuse de réalisation, le réceptacle comporte au moins un puits communiquant avec un micro-canal d'alimentation dont une partie est située en dehors de l'interface d'hydratation, le puits et le micro-canal délimitant un volume calibré de réception pour un échantillon liquide et présentant une section ouverte calibrée d'hydratation délimitée par le bord supérieur du puits et au moins une partie du bord supérieur du micro-canal. Ces micro-canaux permettent d'alimenter plus facilement les puits, par l'échantillon liquide. Après hydratation, les micro-canaux vides permettent de faire circuler l'air dans les puits offrant l'avantage de limiter les effets de condensation au fond des puits pour faciliter la lecture au travers.

Avantageusement, chaque puits présente une section ouverte calibrée d'hydratation délimitée par un contour qui en dehors des micro-canaux, laisse subsister, en position d'hydratation, sur la face d'absorption du support hydratant, une ceinture périphérique entre ledit contour et le bord périphérique du support hydratant. Ainsi, l'échantillon liquide est absorbé sans risque de fuites et d'inter pollution entre les puits.

Selon une variante de réalisation, le couvercle est pourvu de plusieurs supports hydratants destinés à être hydratés chacun par un échantillon liquide et en ce que le nombre et le volume des puits en regard de chaque support hydratant sont identiques pour tous les supports hydratants. Une telle variante de réalisation permet d'hydrater de façon identique et simultanée, plusieurs supports hydratants, ce qui permet une détection multiplexée de microorganismes.

Afin d'améliorer les conditions de transfert de l'échantillon liquide sur le support hydratant, au moins un puits présente un profil qui se rétrécit à partir de son bord supérieur.

Selon une caractéristique avantageuse de réalisation, le réceptacle comporte une plaque dans laquelle le ou les puits et les micro-canaux sont aménagés en étant entourés par un rebord périphérique servant, en position d'hydratation, de surface de butée pour les supports hydratants.

Selon un mode préféré de réalisation, le réceptacle comporte une plaque dans laquelle le ou les puits sont aménagés et à partir de laquelle s'élève en saillie, une bordure périphérique entourant les puits et délimitant intérieurement, au moins une cuvette de répartition pour le ou les échantillons liquides. Un tel réceptacle facilite l'opération de répartition de ou des échantillons liquides en une seule opération sans risque de débordement.

Dans le cas où l'échantillon liquide est transparent, chaque puits est avantageusement pourvu d'un colorant pour l'échantillon liquide afin d'aider au remplissage dudit puits.

Selon un mode de réalisation, le réceptacle comporte au moins une cloison de séparation en vue de délimiter au moins deux cuvettes de répartition chacun pour un échantillon liquide différent d'une cuvette à l'autre. Selon ce mode de réalisation, le dispositif permet d'assurer des conditions différentes d'hydratation, par exemple avec un échantillon liquide de dilution différente permettant de disposer par exemple d'un support hydratant témoin.

Avantageusement, le couvercle possède une rainure périphérique apte à s'emboîter dans la bordure périphérique du réceptacle.

Selon une variante de réalisation, le couvercle et le réceptacle sont ajustés pour obtenir un emboîtement étanche de manière à limiter le passage d'air à l'intérieur du dispositif pour limiter l'évaporation lors de l'incubation et le dessèchement des supports hydratants.

Selon une autre variante de réalisation, le couvercle et le réceptacle sont aménagés de manière qu'en position emboîtée, l'air puisse circuler entre le couvercle et le réceptacle afin d'obtenir une ventilation contrôlée des supports hydratants et favoriser ainsi la croissance microbienne.

Selon une caractéristique de l'invention, le dispositif comporte un système de centrage relatif entre le couvercle et le réceptacle pour positionner chaque support hydratant en position superposée par rapport à un ou plusieurs puits de réception d'un échantillon liquide. Un tel système facilite l'opération d'hydratation consistant à rapprocher le couvercle et le réceptacle afin que chaque support hydratant vienne en contact avec un échantillon liquide et cela de façon simultanée pour l'ensemble des supports hydratants.

Selon une variante avantageuse de réalisation, le système de centrage permet le montage du couvercle sur le réceptacle, sans sens de montage de sorte que le positionnement relatif entre les puits et les supports hydratants est symétrique. Bien entendu, le couvercle et/ou le réceptacle peuvent être équipés d'un détrompeur pour autoriser un seul sens de montage du couvercle sur le réceptacle.

Avantageusement le dispositif selon l'invention comporte un système de verrouillage du couvercle et du réceptacle dans une position stable correspondant à la position d'hydratation. Ce système de verrouillage permet également de ré-ouvrir le dispositif pour faire un prélèvement en vue d'une autre analyse. Le système de verrouillage peut également être conçu pour assurer un assemblage définitif ne permettant pas une réouverture facile. Selon une autre variante de réalisation, le système de verrouillage peut assurer un verrouillage différent en fonction du sens de montage du couvercle sur le réceptacle, à savoir ouvrable facilement dans un sens de montage et ouvrable difficilement pour l'autre sens de montage. Par ailleurs le couvercle peut disposer d'un détrompeur pour avoir qu'un seul sens de verrouillage.

Selon une caractéristique préférée de réalisation, le réceptacle et/ou le couvercle sont réalisés en un matériau transparent ou translucide. Un tel dispositif offre ainsi l'avantage de permettre la lecture directe, sans manipulation, de la réaction obtenue par la mise en contact de l'échantillon liquide avec le support hydratant.

Le dispositif d'hydratation selon l'invention présente une utilisation particulièrement avantageuse pour détecter et/ou identifier et/ou énumérer au moins un microorganisme dans un échantillon liquide susceptible de le contenir.

Une utilisation préférée du dispositif selon l'invention est de permettre de déterminer la sensibilité et la résistance d'au moins un microorganisme à au moins un antibiotique.

Un autre objet de l'invention est de proposer un procédé pour hydrater au moins un support hydratant par un échantillon liquide à l'aide d'un dispositif conforme à l'invention. Afin de détecter et/ou identifier et/ou énumérer au moins un microorganisme cible dans un échantillon liquide susceptible de le contenir, le procédé comporte les étapes suivantes :
- répartir au moins un échantillon liquide dans un ou plusieurs puits du réceptacle ;
- positionner le couvercle et le réceptacle dans leur position d'hydratation dans laquelle chaque support hydratant est en contact avec un échantillon liquide ;
- maintenir le couvercle et le réceptacle en position d'hydratation pour la réalisation d'une étape d'incubation ;
- lire chaque support hydratant afin de détecter et/ou identifier et/ou énumérer ledit au moins un microorganisme cible.

Le procédé selon l'invention permet ainsi d'hydrater facilement et simplement, un support hydratant par un échantillon liquide.

Par « détection », l'on entend la détection à l'œil nu ou à l'aide d'un appareil optique de la présence de microorganismes cibles ou de l'existence d'une croissance de microorganismes cibles.

Lorsque le milieu réactionnel à partir duquel l'on souhaite détecter les microorganismes cibles comprend un substrat chromogène ou fluorogène, la détection peut être effectuée à l'aide d'un appareil optique pour les substrats fluorogènes, ou à l'œil nu ou à l'aide d'un appareil optique pour les substrats chromogènes.

Par « dénombrement », l'on entend le fait de comptabiliser/quantifier le nombre de microorganismes cibles, par exemple le nombre de colonies bactériennes lorsque le microorganisme cible est une bactérie.

Par « incuber », on entend porter et maintenir entre 1h et 48h, préférentiellement entre 4 et 24 heures, à une température appropriée, généralement comprise entre 20°C et 50°C, préférentiellement entre 30 et 45°C.

Selon un mode de mise en œuvre, le procédé consiste à répartir chaque échantillon liquide en versant dans le réceptacle le ou les échantillons liquides et en agitant le réceptacle afin de s'assurer que chaque échantillon liquide remplisse complètement le ou les puits de réception.

Avantageusement, le procédé selon l'invention consiste à réaliser l'étape de lecture de chaque support hydratant à travers le réceptacle et/ou le couvercle. Il est à noter que le procédé offre aussi la possibilité, après l'étape d'incubation, de pouvoir remplacer le réceptacle par un fond plan transparent ou translucide à travers lequel l'opération de lecture de chaque support hydratant est réalisée.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est une vue de dessus d'un dispositif conforme à l'invention pour hydrater un support hydratant par un échantillon liquide.
La **Figure 2** est une vue de côté du dispositif d'hydratation illustré à la **Fig. 1****.**
La **Figure 3** est une vue en coupe élévation partielle du dispositif d'hydratation illustré à la **Fig. 2****.**
La **Figure 4** est une vue en perspective d'un exemple de réalisation d'un réceptacle illustré à la **Fig. 1** et faisant partie du dispositif d'hydratation conforme à l'invention.
La **Figure 5** est une vue de dessus de détail du réceptacle illustré à la **Fig. 4****.**
La **Figure 6** est une vue en coupe prise selon les lignes VI-VI de la **Fig. 5** et montrant en détail la forme des puits.
La **Figure 7** est une vue de dessus d'un autre exemple de réalisation d'un réceptacle comportant des puits de réception de forme oblongue inclinée.
La **Figure 8** illustre un autre exemple de réalisation d'un réceptacle comportant des puits de réception de forme oblongue orientés selon un côté du réceptacle.
La **Figure 9** illustre une vue de dessus d'un autre exemple de réalisation d'un réceptacle muni de puits de forme carrée assurant chacun la réception d'un échantillon liquide.
La **Figure 10** illustre une vue de dessus d'un autre exemple de réalisation d'un réceptacle muni de puits de forme carrée groupés par quatre de sorte que chaque groupe de puits assure la réception d'un échantillon liquide.
La **Figure 11** est une vue de dessus d'un exemple de réalisation d'un puits de réception comportant quatre cavités de section circulaire communiquant entre-elles par leurs fonds.
La **Figure 12** est une vue de dessus d'un exemple de réalisation d'un puits de réception comportant quatre cavités de section circulaire communiquant entre-elles au niveau de leurs bords supérieurs.
La **Figure 13** est une vue de dessus d'un autre exemple de réalisation d'un puits de réception comportant quatre cavités de section carrée communiquant entre-elles dans une zone centrale au niveau de leurs bords supérieurs.
La **Figure 14** est une vue de dessus d'un autre exemple de réalisation d'un puits de réception comportant quatre cavités de section carrée communiquant entre-elles deux à deux au niveau de leurs bords supérieurs.
La **Figure 15** est une vue en perspective d'un autre exemple de réalisation d'un réceptacle pourvu de puits présentant chacun plusieurs cavités au centre duquel un îlot est aménagé.
La **Figure 15A** est une vue de dessus d'un puits aménagé dans le réceptacle illustré à la **Fig. 15****.**
La **Figure 16** est une vue en perspective d'un autre exemple de réalisation d'un dispositif d'hydratation conforme à l'invention dont le réceptacle comporte des puits de réception comprenant chacun deux cavités reliées entre elles.
La **Figure 17A** est une vue en perspective illustrant un réceptacle dont les puits sont remplis par des échantillons liquides.
La **Figure 17B** est une coupe élévation montrant l'opération d'hydratation au cours de laquelle le support hydratant est placé en contact avec l'échantillon liquide.
La **Figure 17C** est une vue en coupe élévation montrant le dispositif au terme de l'opération d'hydratation des supports hydratants par les échantillons liquides.

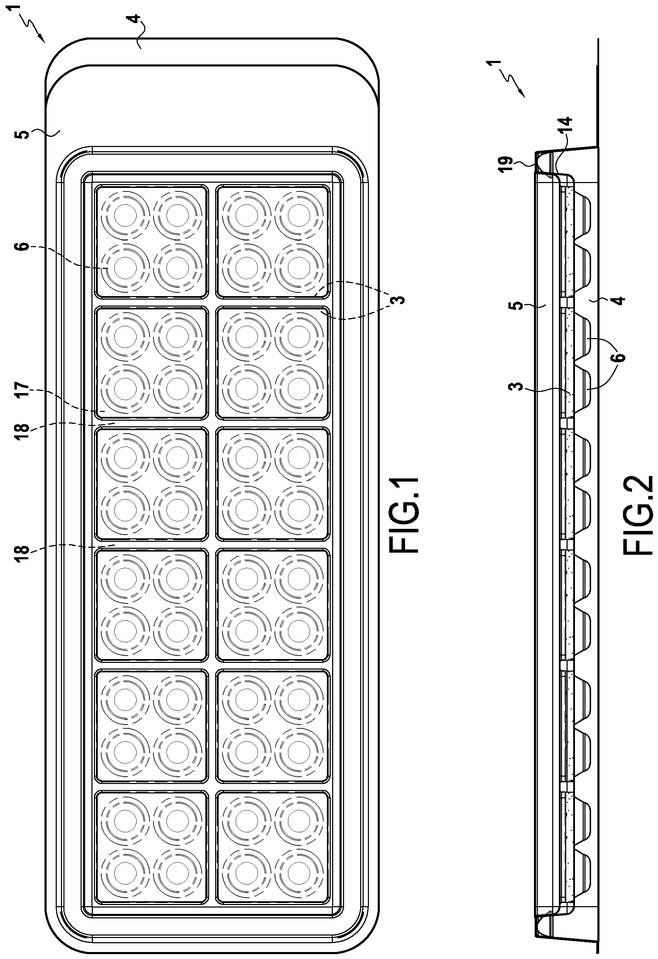

Tel que cela ressort plus précisément des **Fig. 1** à **6****,** l'objet de l'invention concerne un dispositif **1** pour hydrater, par un échantillon liquide **2,** un support hydratant **3** comprenant un milieu réactionnel adapté à l'échantillon liquide **2.** Le dispositif **1** selon l'invention comporte ainsi un réceptacle **4** de réception pour au moins un échantillon liquide **2** et un couvercle **5** équipé d'au moins d'un support hydratant **3.** Comme cela sera décrit en détail dans la suite de la description, le dispositif **1** est destiné à occuper plusieurs positions à savoir, une position d'attente d'hydratation dans laquelle le réceptacle **4** ne contient pas encore l'échantillon liquide **2** et une position d'hydratation du ou des supports hydratants **3.**

Pour cette position d'hydratation, après le versement du ou des échantillons liquides **2** dans le réceptacle **4,** le couvercle **5** est placé en position superposée par rapport au réceptacle **4** pour assurer la mise en contact de chaque support hydratant **3** avec un échantillon liquide **2.** La zone de contact entre le support hydratant **3** et l'échantillon liquide **2** est appelée interface d'hydratation dans la suite de la description. Selon un mode avantageux de mise en œuvre, le réceptacle **4** et le couvercle **5** sont maintenus en position fermée de sorte que le dispositif **1** occupe une position d'incubation au terme de laquelle une opération de lecture de chaque support hydratant peut être réalisée.

Le dispositif **1** selon l'invention comportant le réceptacle **4** et le couvercle **5** se présente ainsi sous la forme d'un kit pouvant être utilisé facilement pour hydrater un support hydratant **3.** Dans les exemples illustrés sur les dessins, le dispositif **1** présente une forme rectangulaire avec deux côtés opposés de petites dimensions et deux autres côtés opposés de plus grandes dimensions. Le dispositif **1** selon l'invention trouve des utilisations dans de nombreux domaines d'applications.

Par exemple, le dispositif **1** selon l'invention peut être utilisé pour permettre une hydratation d'un support hydratant qui nécessite juste avant utilisation, une réhydratation pour activer le milieu réactionnel du support hydratant afin de rendre prêt à l'emploi, le support hydraté et imprégné au préalable du milieu réactionnel.

Le dispositif **1** selon l'invention permet également d'hydrater un support hydratant imprégné d'un milieu réactionnel afin de pouvoir après incubation, réaliser un contrôle microbiologique.

Bien entendu, le milieu réactionnel est choisi en fonction de l'application et de l'échantillon liquide utilisé et de l'analyte et/ou des microorganismes à détecter ou identifiés et/ou à énumérer.

Selon la présente invention, l'échantillon biologique peut être de diverses origines, par exemple d'origine alimentaire, environnementale (eau), vétérinaire clinique, cosmétique ou pharmaceutique.

Les échantillons biologiques d'origine clinique peuvent correspondre à des prélèvements de fluides biologiques (sang total, sérum, plasma, urine, liquide céphalo-rachidien, etc.), de selles, de prélèvements de nez, de gorge, de peau, de plaies, d'organes, de tissus ou de cellules isolées. Cette liste n'est évidemment pas exhaustive.

D'une manière générale, le terme « échantillon » se réfère à une partie ou à une quantité (plus particulièrement une petite partie ou une petite quantité) prélevées à partir d'une ou plusieurs entités aux fins d'analyse. Cet échantillon peut éventuellement avoir subi un traitement préalable, impliquant par exemple des étapes de mélange, de dilution ou encore de broyage, en particulier si l'entité de départ est à l'état solide.

L'échantillon biologique prélevé est, en général, susceptible de - ou suspecté de - contenir au moins un micro-organisme cible. Dans la plupart des cas, ce dernier est un micro-organisme pathogène (tel que Salmonella ou *Vibrio cholerae*) qu'il convient de détecter à des fins sanitaires. Ce micro-organisme cible peut également consister en une bactérie résistante aux antibiotiques (antibio-résistante).

De par sa nature et ses applications, la présente invention est particulièrement adaptée à l'analyse d'échantillons biologiques potentiellement à pouvoir pathogène modéré à important (voire même extrêmement important), tels qu'un prélèvement de diarrhée cholérique.

Au sens de la présente invention, le terme micro-organisme recouvre les bactéries à Gram positif ou Gram négatif, les levures, les moisissures, les amibes et plus généralement, les organismes unicellulaires, invisibles à l'œil nu, qui peuvent être manipulés et multipliés en laboratoire.

Selon un mode préféré de réalisation de l'invention, le micro-organisme est une bactérie, gram négative ou positive.

A titre de bactéries Gram positives, on peut citer les bactéries des genres suivants : *Enterococcus, Streptococcus, Lactobacillus, Bifidobacterium, Staphylococcus, Bacillus, Listeria, Clostridium, Mycobacteria, Nocardia, Corynebacteria, Micrococcus et Deinococcus.*

A titre de bactéries Gram négatives on peut citer les bactéries des genres suivants : *Salmonella, Escherichia coli, Pseudomonas, Vibrio cholerae.*

A titre de levures, on peut citer les levures des genres suivants : *Candida, Cryptococcus, Saccharomyces et Trichosporon.*

*A titre de moisissures, on peut citer les moisissures des genres suivants : Aspergillus, Penicillium, Cladosporium.*

La sélection et le pré traitement des échantillons liquides, de nature biologique, industrielle ou environnementale, sont des opérations bien connues dans l'état de la technique et ne sont pas décrites plus en détail. De même, le milieu réactionnel imprégnant le support hydratant **3** peut être de toute nature appropriée en fonction de l'application visée. Par exemple, le milieu réactionnel peut être un milieu de culture chromo génique permettant aux espèces microbiennes recherchées d'avoir une couleur spécifique ou un milieu uniquement de révélation des analytes d'intérêt sans apport nutritionnel car c'est le liquide à analyser qui apporte les nutriments requis pour la physiologie microbienne (cas du lait).

Par milieu réactionnel, on entend un milieu comprenant tous les éléments nécessaires à la survie et/ou à la croissance des microorganismes. Ce milieu réactionnel peut soit servir uniquement de milieu de révélation, soit servir de milieu de culture et de révélation. Dans le premier cas, la culture des microorganismes a lieu préalablement, et, dans le second cas, le milieu réactionnel constitue également le milieu de culture.

On entend par milieu de révélation, tout milieu contenant une molécule capable de se coupler avec les micro-organismes ou les partenaires de liaison desdits micro-organismes et permettant, par leurs propriétés de transduction (fluorescence, coloration, radioactivité, etc.) de révéler la présence desdits micro-organismes. Cette révélation de la présence des micro-organismes cibles peut être notamment obtenue par visualisation (à l'œil nu) ou lecture optique d'une coloration ou d'une fluorescence sur tout ou partie du support.

On entend par milieu de culture, un milieu comprenant tous les éléments nécessaires à la survie et/ou à la croissance de micro-organismes. En pratique, l'homme du métier choisira le milieu de culture en fonction des micro-organismes cibles, selon des critères parfaitement connus et à la portée de cet homme de l'art.

Le milieu réactionnel selon l'invention peut contenir d'éventuels additifs comme par exemple : des peptones, un ou plusieurs facteurs de croissance, des hydrates de carbone, un ou plusieurs agents sélectifs, des tampons, colorants, un ou plusieurs gélifiants, des hydrogels, agent visqueux, etc.

Un tel milieu réactionnel imprègne de toute manière appropriée connue de l'homme du métier, le support hydratant **3.** Ainsi, le support hydratant peut être mis en solution dans l'eau ou dans un solvant puis séché. Le support hydratant peut également être imprégné par un milieu réactionnel déshydraté tel que décrit dans la demande de brevet internationale PCT/FR2015/050035 déposée par la demanderesse. Le support hydratant **3** est réalisé de toute manière appropriée pour intégrer et conserver le milieu réactionnel en son sein, pour assurer son hydratation ultérieure, par un échantillon liquide. A cet effet, le support hydratant **3** est réalisé à base de divers composés absorbants à très fort pouvoir de rétention d'eau tels que la viscose, rayonne, le coton, les fibres cellulosiques naturelles ou modifiées chimiquement comme la carboxy-méthyl cellulose, les polymères chimiques absorbants ou super-absorbants tels que sels de polyacrylate, copolymère acrylate/acrylamide. Le support hydratant a un volume à porosité adaptée, sous forme de tissus ou de non-tissé présentant un réseau fibreux ou filamenteux, de mousse à porosité ouverte.

Le réceptacle **4** du dispositif selon l'invention est adapté pour recevoir un ou plusieurs échantillons liquides **2,** fonction du nombre de supports hydratants **3** à hydrater. Dans les exemples illustrés sur les dessins, le réceptacle **4** est adapté pour recevoir plusieurs échantillons liquides **2** destinés à être mis en contact chacun avec un support hydratant **3.** Dans les exemples illustrés sur les différentes **Figures,** le couvercle **5** est pourvu de douze supports hydratants **3** de sorte que le réceptacle **4** est destiné à recevoir douze échantillons liquides **2.** Bien entendu, le couvercle **5** peut comporter un nombre différent de supports hydratants **3.** De même, le réceptacle **4** peut recevoir un nombre différent d'échantillons liquides.

Chaque échantillon liquide **2** est destiné à être contenu dans un ou plusieurs puits **6** aménagés dans le réceptacle **4.** Le réceptacle **4** est donc configuré en fonction du nombre de puits **6** choisis pour accueillir chaque échantillon liquide et du nombre d'échantillons liquides utilisés qui est égal généralement au nombre de supports hydratants **3,** même si il peut être envisagé de ne pas hydrater un ou plusieurs de supports hydratants **3** du réceptacle **4.** Le ou les puits **6** de réception d'un échantillon liquide **2** sont donc aménagés pour être situés en regard ou en vis à vis du support hydratant **3** devant être hydraté par ledit échantillon liquide.

En position de réception d'un échantillon liquide, le réceptacle **4** est positionné dans un plan horizontal de manière que chaque puits **6** se trouve ouvert vers le haut pour récupérer le liquide. Chaque puits **6** comporte ainsi un orifice ou une ouverture d'entrée **7** au niveau d'un bord supérieur **8** du puits à partir duquel s'étend vers le bas au moins une cavité **6₁**. Par définition, il est considéré que chaque puits **6** possède un orifice d'entrée **7** dont le contour **C** formé par le bord supérieur **8** est fermé c'est à dire se reboucle sur lui-même.

Dans l'exemple illustré, le réceptacle **4** comporte une plaque **9** dans laquelle le ou les puits **6** sont aménagés pour s'étendre en creux. Chaque puits **6** comporte ainsi au moins une cavité **6₁** présentant une paroi **10** s'étendant sur une hauteur limitée entre un fond fermé **11** et un rebord périphérique **12** entourant le bord supérieur **8** du puits. La paroi **10** s'étend en dessous ou en retrait par rapport au rebord périphérique **12** qui comme cela sera compris dans la suite de la description, sert de surface de butée et d'appui pour les supports hydratants **3** en position d'hydratation. Chaque puits **6** possède ainsi une contenance ou un volume calibré défini jusqu'au niveau de son bord supérieur **8** et qui est vide avant son remplissage par l'échantillon liquide. De même, chaque puits **6** possède au niveau de son orifice **7** une superficie, une surface ou une aire calibrée de manière précise.

Selon une caractéristique avantageuse de réalisation, le réceptacle **4** est aménagé pour délimiter au moins une cuvette **13** de répartition pour le ou les échantillons liquides dans le ou les puits **6.** A cet effet, le réceptacle **4** comporte une bordure périphérique **14** s'élevant à partir de la plaque **9** en entourant l'ensemble des puits **6** pour délimiter intérieurement la cuvette **13.** Tel que cela ressort des **Figures,** le réceptacle **4** possède ainsi en périphérie, une bordure périphérique **14** s'élevant en saillie à partir des rebords périphériques **12** bordant les différents puits, ces rebords **12** s'étendant dans un plan en dessous duquel sont situés le ou les puits **6.** Cette bordure périphérique **14** permet de confiner l'échantillon liquide sans risque de débordement, lors de l'opération de remplissage des puits.

La répartition des échantillons liquides **2** à l'intérieur des puits **6** est réalisée de manière simple puisqu'il suffit de verser à l'intérieur de la cuvette **13** le volume total correspondant aux échantillons liquides à répartir dans les différents puits de manière que le liquide s'établisse dans les différents puits.

Cette répartition peut éventuellement être favorisée par une phase d'agitation du réceptacle **4** afin de s'assurer que chaque échantillon liquide remplisse complètement les puits de réception prévus. Dans le cas d'un échantillon transparent, chaque puits **6** est pourvu d'un colorant pour l'échantillon liquide afin d'aider au remplissage dudit puits.

Selon une autre caractéristique avantageuse de réalisation, au moins certains des puits **6** sont pourvus de micro-canaux **16** pour faciliter l'alimentation des puits par l'échantillon liquide.

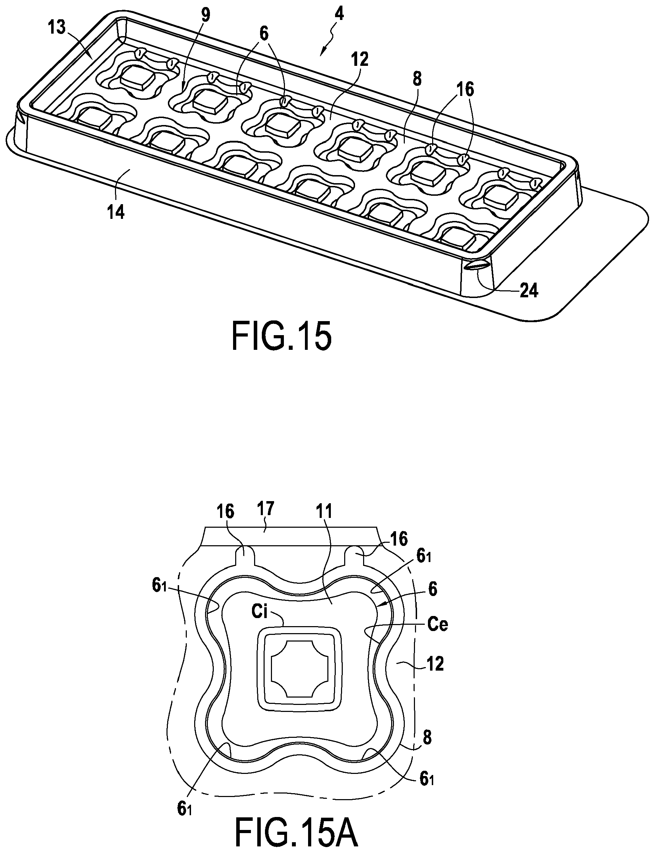

Avantageusement, tous les puits **6** voisins de la bordure périphérique **14** sont pourvus de micro-canaux **16.** Ainsi, tel que cela ressort de la **Fig. 15****,** chaque puits **6** voisin de la bordure périphérique **14** comporte au moins un et dans l'exemple, deux micro-canaux **16** aménagés dans la plaque **9** du réceptacle **4** pour déboucher au niveau du bord supérieur **8** du puits **6.** Chaque micro-canal **16** comporte ainsi un bord supérieur **8** présentant une continuité avec le bord supérieur **8** proprement dit du puits **6** de sorte que le contour **C** du puits incluant le ou les micro-canaux **16** est fermé. Il est à considérer que le puits **6** et le micro-canal **16** délimitent un volume calibré de réception pour un échantillon liquide **2** en considérant que chaque micro-canal est fermé à son extrémité opposée de celle communiquant avec le puits. De même, chaque micro-canal **16** présente au niveau de son bord supérieur **8,** une superficie, une surface ou une aire calibrée de manière précise.

Avantageusement, chaque micro-canal **16** s'étend à partir du bord supérieur **8** du puits en direction de la bordure périphérique **14** et de préférence, à l'aplomb de la bordure périphérique **14** de manière que lorsque le liquide circule le long de la bordure périphérique, le liquide est récupéré par les micro-canaux **16** pour l'acheminer dans les puits **6.**

Ainsi, les micro-canaux **16** sont situés au moins en partie en dehors de l'interface d'hydratation de sorte qu'en position d'hydratation, les micro-canaux **16** débordent par rapport au support hydratant **3.** Typiquement, chaque micro-canal **16** déborde d'au moins 1 mm par rapport au support hydratant **3.** Outre le fait que ces micro-canaux facilitent la répartition des échantillons liquides dans les puits, les micro-canaux **16** permettent également après l'opération d'hydratation, de favoriser l'échange d'air à l'intérieur du dispositif **1.**

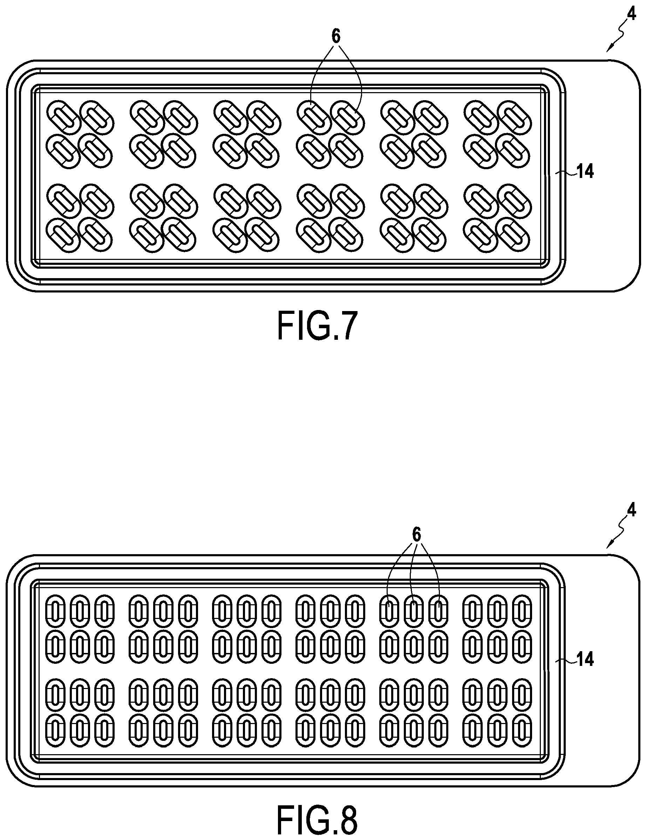

Dans l'exemple de réalisation illustré aux **Fig. 1****,** **7** et **10****,** chaque échantillon liquide **2** est réparti dans un groupe de quatre puits **6** de sorte que le réceptacle **4** qui peut recevoir douze échantillons liquides **2,** comporte quarante-huit puits **6.** Dans les exemples de réalisation illustrés respectivement aux **Fig. 8****,** **9** et **16** le nombre de puits **6** formant un groupe de réception d'un échantillon liquide est respectivement de six, d'un et de deux Dans ces exemples de réalisation illustrés aux **Fig. 8****,** **9** et **16****,** le réceptacle **4** peut recevoir douze échantillons liquides **2.**

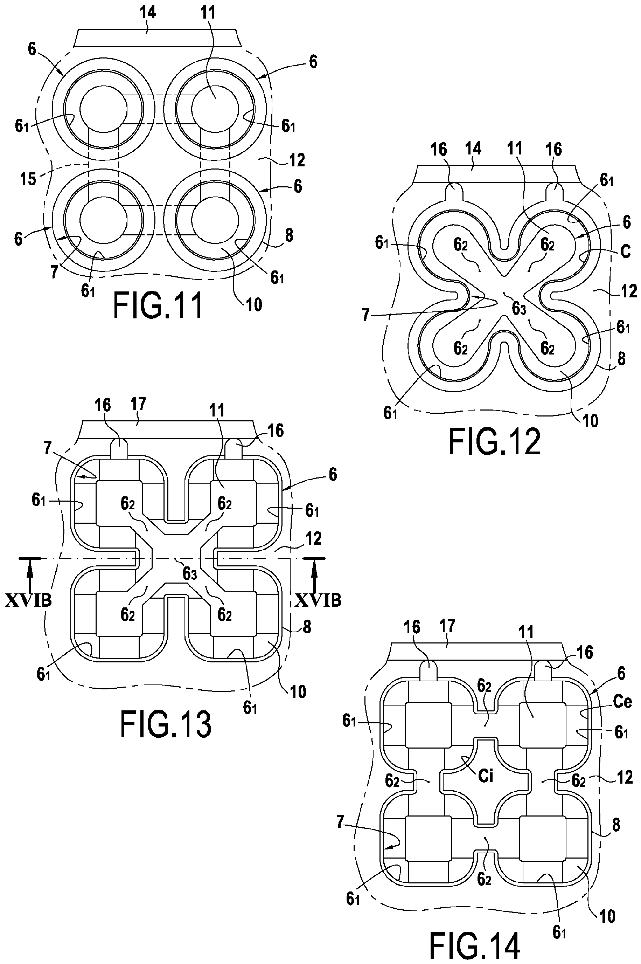

Bien entendu, le nombre de puits **6** pour recevoir un échantillon liquide et par suite, hydrater un support hydratant **3,** peut être différent des exemples représentés. Par ailleurs, dans les exemples illustrés aux **Fig. 1****,** **7****,** **8** et **10****,** les puits **6** appartenant à un même groupe de réception d'un échantillon liquide sont indépendants les uns des autres dans la mesure où aucun des puits appartenant à un même groupe ne communique avec un autre puits. Cependant, il peut être prévu que les cavités **6₁** des puits **6** de réception d'un échantillon liquide communiquent entre elles par des conduits de liaison **15** alors que chaque puits **6** conserve un contour fermé au niveau de son bord supérieur **8.** Comme cela est illustré à la **Fig. 11****,** les conduits de liaison **15** relient deux à deux les fonds **11** des cavités **6₁** des quatre puits qui sont destinés à recevoir ensemble un échantillon liquide.

Bien entendu, les puits **6** peuvent recevoir différentes formes possibles adaptées pour assurer l'hydratation du support hydratant **3.** Ainsi, comme cela apparaît sur les Figures, les puits **6** peuvent présenter une forme par exemple hémisphérique, oblongue, conique ou prismatique.

Dans le même sens, l'ouverture d'entrée **7** des puits **6** peut présenter différentes formes pour assurer une hydratation homogène des supports hydratants **3.** Ainsi, l'ouverture d'entrée **7** des puits **6** peut présenter une forme ronde **(****Fig. 1****),** oblongue (**Fig. 7, 8****)** ou carrée **(****Fig. 9, 10****).**

Les **Fig. 12** à **16** illustrent à titre d'exemple diverses autres formes de réalisation pour l'ouverture d'entrée **7** des puits **6.** Selon ces exemples, l'ouverture d'entrée **7** de chaque puits **6** possède un contour fermé **C** présentant différents lobes de profils diversifiés en arc de cercle ou autres. Dans les exemples illustrés aux **Fig. 12** et **13****,** chaque puits **6** comporte quatre cavités **6₁** disposées aux quatre coins d'un carré, en étant légèrement écartées les unes des autres. Ces quatre cavités **6₁** sont reliées entre elles au niveau du centre de ce carré, de manière que le puits **6** possède un orifice unique d'entrée **7** délimité par un bord supérieur **8** continu se refermant sur lui-même. L'orifice **7** du puits **6** présente ainsi une forme de trèfle.

Dans l'exemple de réalisation illustré à la **Fig. 12****,** les quatre cavités **6₁** possèdent chacune une section en forme de 3/4 d'un cercle et communiquent entre elles par des passages de communication **6₂** débouchant dans une zone centrale **6₃** de l'orifice **7**. Par exemple, chaque passage de communication **6₂** possède une largeur supérieure au rayon du cercle délimitant la cavité mais inférieure au diamètre dudit cercle.

Dans l'exemple illustré à la **Fig. 13****,** les quatre cavités **6₁** possèdent chacune une section carrée et communiquent entre elles par des passages de communication **6₂** aménagés dans les coins voisins desdites cavités pour déboucher dans une zone centrale **6₃** de l'orifice **7,** présentant une surface du même ordre que la surface délimitée par une section carrée d'une cavité **6₁**.

Dans l'exemple de réalisation illustré à la **Fig. 14****,** les quatre cavités **6₁** sont également de section carrée et disposées aux quatre coins d'un carré mais ces cavités **6₁** communiquent entre elles deux à deux par des passages de communication **6₂**. Ainsi, l'orifice **7** présente un contour externe fermé **Cₑ** et un contour interne fermé **Cᵢ** formant un îlot au centre de l'orifice **7.** Bien entendu, la section ouverte d'hydratation du puits **6** est située entre les contours externe **Cₑ** et interne **Cᵢ**.

Les **Fig. 15** et **15A** illustrent un autre exemple de réalisation sensiblement analogue à l'exemple illustré à la **Fig. 14** à la différence que les cavités **6₁** sont de section circulaire et sont raccordées entre elles par des profils courbes. Selon cet exemple, l'orifice **7** du puits **6** présente un contour fermé externe **Cₑ** et un contour interne fermé **Cᵢ** formant un îlot au centre de l'orifice **7.** Dans les exemples décrits en relation avec les **Fig. 12** à **15****,** chaque puits **6** comporte 4 cavités **6₁** reliées entre elles. Bien entendu, le nombre de cavités formant un puits peut être différent de quatre.

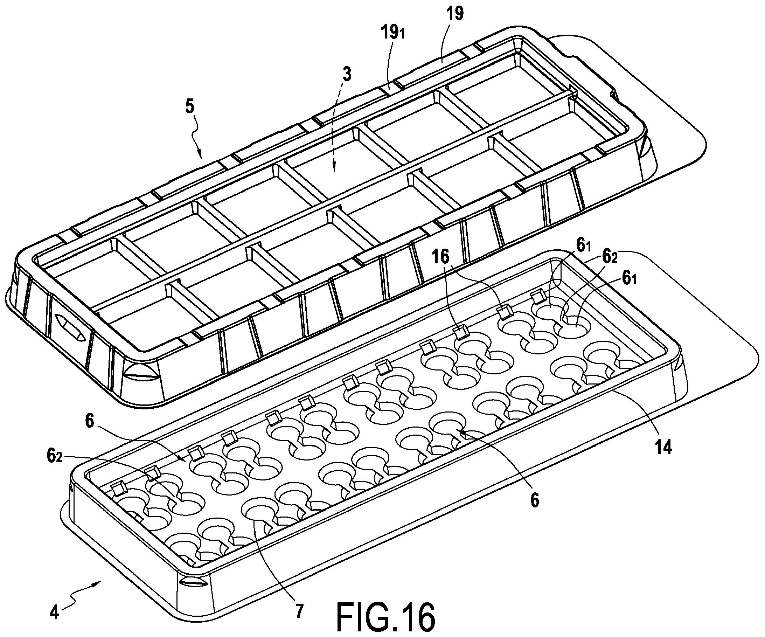

La **Fig. 16** illustre un autre exemple de réalisation sensiblement analogue à l'exemple de réalisation illustré à la **Fig. 12** à la différence que les quatre cavités **6₁** de réception d'un échantillon liquide se répartissent par paires. Chaque puits **6** comporte deux cavités **6₁** reliées entre elles. Les deux cavités **6₁** d'une paire communiquent entre elles par un passage de communication **6₂**. Ainsi, l'orifice **7** d'un puits **6** présente une forme générale oblongue puisqu'elle présente à chaque extrémité, une section en forme de ¾ d'un cercle et dans la partie centrale, un passage de communication **6₂** de section rectiligne de largeur inférieure au diamètre de la section en forme de ¾ de cercle.

Avantageusement, ce passage de communication **6₂** est centré par rapport aux sections en forme de ¾ de cercle.

Il est à noter que selon cette variante de réalisation, tous les puits **6** sont pourvus de micro-canaux **16.** Plus précisément, la cavité **6₁** de chaque puits le plus proche de la bordure périphérique **14** est pourvue d'un micro-canal **16** s'étendant en direction de cette bordure périphérique **14.**

Avantageusement, l'orifice **7** d'un puits **6** est aménagé afin que la forme oblongue se trouve orientée parallèlement au côté du réceptacle présentant la plus petite dimension. Le basculement du réceptacle lors de l'opération de répartition de l'échantillon liquide dans les puits, selon le côté du réceptacle présentant la plus grande dimension permet une bonne répartition du liquide dans les puits.

Selon une caractéristique avantageuse de réalisation, au moins un puits **6** présente un profil qui se rétrécit à partir de son bord supérieur **8.** Ainsi, tel que cela ressort plus précisément des **Fig. 3** et **6****,** la paroi **10** du puits **6** s'étend à partir du rebord **12** selon un angle obtus de sorte que le puits présente une forme évasée en direction de son bord supérieur **8.** Le profil du puits **6** qui se rétrécit en direction de son fond, peut être limité sur une partie de la hauteur de la paroi **10** ou être continu, entre le bord supérieur et le fond du puits. Selon une autre variante de réalisation, il peut être envisagé que la paroi **10** s'étende sensiblement à l'équerre par rapport au rebord périphérique **12** en étant pourvu ou non, d'une forme évasée au niveau de son bord supérieur **8.**

Comme déjà expliqué, chaque échantillon liquide **2** est reçu par un ou plusieurs puits de réception **6.** Dans l'exemple illustré aux **Fig. 1****,** **7****,** **10****,** **11** chaque échantillon liquide **2** est reçu par quatre puits **6** alors que dans l'exemple illustré à la **Fig. 16****,** chaque échantillon liquide **2** est reçu par deux puits **6.** En d'autres termes, le volume total d'un échantillon du liquide **2** est réparti selon ces exemples, soit dans les quatre puits **6** attribués, soit dans deux puits **6** voire également dans les micro-canaux **16** qui les alimentent. Chaque échantillon liquide **2** est destiné à être absorbé par un support hydratant **3** lorsque le couvercle **5** et le réceptacle **4** sont placés en position d'hydratation.

A cet effet, le couvercle **5** possède une face inférieure **5₁** sur laquelle au moins un support hydratant **3** est fixé pour présenter une face d'absorption **3₁** pour un échantillon liquide **2.** Cette face d'absorption **3₁** est destinée à être positionnée au-dessus du réceptacle **4** pour venir en contact avec l'échantillon liquide **2** selon l'interface d'hydratation. La fixation des supports hydratants **3** sur le couvercle **5** est réalisée à l'aide de tout système de fixation connu. Par exemple, chaque support hydratant **3** peut être fixé à l'aide d'un procédé de soudure ou à l'aide d'un film adhésif ou de la colle interposé entre le couvercle **5** et la face du support hydratant **3,** opposée de la face d'absorption **3₁**. Pour faciliter le montage, la face inférieure **5₁** du couvercle peut présenter des logements dans lesquels les supports hydratants **3** sont partiellement encastrés.

Conformément à une caractéristique de l'invention, chaque support hydratant **3** présente une face d'absorption **3₁** dont la surface, la superficie ou l'aire est supérieure à la surface, la superficie ou l'aire de la section ouverte calibrée du réceptacle **4** qui permet l'hydratation du support hydratant par un échantillon liquide **2.** Cette section calibrée d'hydratation présentée par le réceptacle **4** correspond à la surface du liquide de chaque échantillon, destinée à être en contact avec le support hydratant en position d'hydratation et située au niveau du bord supérieur **8** du ou des puits **6** et éventuellement également du ou des micro-canaux **16.** Il est à noter que cette section calibrée d'hydratation ne correspond pas nécessairement à la section ouverte calibrée des puits **6** et des micro-canaux **16** dans la mesure où comme cela sera expliqué dans la suite de la description, toute cette section n'est pas nécessairement en contact avec la face d'absorption **3₁** du support hydratant **3.**

Dans le cas où un échantillon liquide **2** est reçu par un unique puits **6,** la surface d'hydratation de la section ouverte calibrée du réceptacle pour un échantillon liquide correspond à la surface de la section ouverte calibrée du puits **6,** cette section ouverte étant délimitée par l'orifice **7** du puits **6.** Il est à noter que ce puits **6** peut comporter une ou plusieurs cavités **6₁**.

Dans le cas où un échantillon liquide **2** est réparti entre plusieurs puits **6**, la surface d'hydratation de la section ouverte calibrée de réception d'un échantillon liquide correspond à la somme des sections ouvertes calibrées des différents puits **6,** ces sections ouvertes étant délimitées par les orifices **7** des différents puits **6.** Il est à noter que ces puits **6** peuvent comporter chacun une ou plusieurs cavités **6₁** indépendantes ou reliées entre elles par leurs fonds.

Lorsqu'un puits **6** est pourvu d'au moins un micro-canal d'alimentation **16,** le puits **6** et le micro-canal **16** délimitent un volume calibré de réception pour un échantillon liquide résultant de l'addition des volumes du puits et du micro-canal. Le puits **6** et le micro-canal **16** présentent une section ouverte calibrée d'hydratation délimitée par le bord supérieur **8** du puits **6** et par le bord supérieur **8** d'au moins une partie du micro-canal **16** destinée à être en contact avec la face d'absorption **3₁** du support. Bien entendu, la partie du micro-canal **16** située en dehors de la face d'absorption **3₁** du support hydratant ne fait pas partie de cette section ouverte calibrée d'hydratation.

Selon une caractéristique de réalisation, la surface de la section ouverte calibrée permettant l'hydratation d'un support hydratant **3** par un échantillon liquide est telle que le ratio de cette surface de la section ouverte calibrée d'hydratation d'un support hydratant par un échantillon liquide sur la surface de la face d'absorption **3₁** du support hydratant **3** est compris entre 40 et 80%, de préférence entre 50 et 75 % et avantageusement encore entre 55 et 65 %. En d'autres termes, la surface de la face d'absorption **3₁** du support hydratant **3** est destinée à être en contact direct avec l'échantillon liquide sur sensiblement les 2/3 de sa surface. Par exemple, la face d'absorption **3₁** de chaque support hydratant **3** possède une surface de 400 mm² et la superficie de la section ouverte d'hydratation délimitée par le puits **6** dont le contour correspond respectivement aux profils des exemples de réalisation illustrés aux **Fig. 12, 13, 14** et **15** est de 278 mm², 260 mm², 266 mm² et 270 mm². De même, la superficie de la section ouverte d'hydratation délimitée par les deux puits **6** de l'exemple illustré à la **Fig. 16** est de 222 mm² pour une surface de 400 mm² pour la face d'absorption **3₁**. Ainsi, le ratio de la surface d'hydratation présenté par un puits sur la surface d'absorption **3₁** est respectivement de 70 %, 65 %, 67 %, 68 % et 55 % pour les exemples illustrés aux **Fig. 12, 13, 14****,** **15** et **16**

Il est à noter que la forme et les dimensions de la section ouverte des puits **6₁** délimitée par les orifices **7** ainsi que le positionnement relatif entre les orifices **7** par rapport aux supports hydratants **3** sont choisis pour optimiser l'hydratation des supports hydratants **3** de manière notamment à ne pas faire apparaître de front de migration liquidienne à l'intérieur des supports hydratants **3.** Ainsi, les orifices **7** possèdent une forme circulaire, oblongue, carré dans les exemples illustrés respectivement aux **Fig. 1****,** **7** et **10****.** Par ailleurs, dans l'exemple illustré à la **Fig. 8****,** les orifices **7** de forme oblongue sont aménagés pour être orientés parallèlement à un bord du support hydratant alors que dans l'exemple illustré à la **Fig. 7****,** les orifices **7** de forme oblongue sont aménagés pour être orientés en oblique par rapport à un bord du support hydratant.

Selon les exemples de réalisation illustrés aux **Fig. 12** à **16****,** les orifices **7** des puits **6** sont aménagés selon une forme limitant la migration transversale du liquide à l'intérieur du support hydratant. A cet effet, chaque puits **6** présente une section ouverte calibrée d'hydratation délimitée par un contour **C, Ce, Ci** dont la forme est choisie de manière qu'aucun des points pris entre l'extérieur de ladite section et le contour de la face d'absorption **3₁**, n'est situé à une distance linéaire supérieure de 3 mm par rapport aux contours les plus proches de ladite face ou de ladite section. En d'autres termes, la forme de l'orifice **7** est choisie de manière à répartir de manière la plus homogène possible, l'interface d'hydratation sur la face d'absorption **3₁** du support hydratant. Ainsi, la distance à parcourir par le liquide pour hydrater la totalité du support hydratant est limitée à une valeur maximale pour éviter la création d'un front de migration de liquide. De plus, la distance à parcourir pour ce liquide est sensiblement identique en tous points à partir du contour **C** des puits. Il doit être compris que chaque support hydratant **3** présente une surface qui recouvre complètement par sa face d'absorption **3₁**, une section ouverte calibrée d'hydratation du réceptacle. Ainsi, par exemple, dans le cas où l'échantillon liquide **2** est réparti dans quatre puits **6,** chaque support hydratant **3** recouvre, en position d'hydratation, complétement par sa face d'absorption **3₁**, les quatre orifices **7** des quatre puits.

Avantageusement, chaque support hydratant **3** recouvre complètement une section ouverte calibrée de réception d'un échantillon liquide **2,** en laissant subsister sur sa face d'absorption **3₁** entre ladite section et le bord périphérique **3₂** du support hydratant, une ceinture périphérique **17** qui en position d'hydratation et en dehors des micro-canaux **16**, n'est pas en contact avec la section ouverte du ou des puits. Ainsi, chaque support hydratant **3** est positionné, en position d'hydratation, de manière que l'orifice **7** du ou des puits **6** se trouvent positionnés en dehors de cette ceinture périphérique **17** c'est-à-dire en retrait du bord périphérique **3₂** afin que le liquide des puits et des micro-canaux se trouve absorbé totalement par le support hydratant sans risque de fuites selon les bords du support hydratant.

Il ressort de la description qui précède que le profil ou la forme de la section ouverte calibrée d'hydratation délimitée par les puits (voire également par les micro-canaux) est choisie pour éviter la formation d'un front de migration de liquide dans le support hydratant. Par ailleurs, la forme et/ou les dimensions des puits **6** sont choisies pour permettre de calibrer précisément l'échantillon liquide réceptionné par de tels puits. Avantageusement, chaque puits **6** comporte plusieurs cavités **6₁** de réception pour une partie de l'échantillon liquide, ces cavités de réception communiquant entre-elles au moins au niveau de la section ouverte calibrée d'hydratation dont le contour **C** possède différents lobes. Ainsi, chaque puits **6** présente une section ouverte calibrée d'hydratation délimitée par un contour dont la forme est choisie de manière qu'aucun des points pris à l'intérieur de ladite section, n'est situé à une distance linéaire supérieure de 4 mm par rapport au contour le plus proche de ladite section. Une telle caractéristique évite de réaliser des cavités de réception avec des dimensions trop grandes ne permettant pas un dosage précis de l'échantillon.

De manière complémentaire, chaque puits **6** présente une section ouverte calibrée d'hydratation délimitée par un contour **C** dont la forme est choisie de manière que la plus petite dimension de cette section soit supérieure à 1,5 mm. En d'autres termes, une telle caractéristique évite de réaliser des cavités de réception **6₁** avec des dimensions trop petites entrainant une difficulté pour le remplissage des puits par l'échantillon.

Selon une caractéristique avantageuse, il doit être considéré que les puits de réception **6** incluant éventuellement les micro-canaux **16** d'alimentation délimitent un volume calibré afin qu'en position d'hydratation, chaque échantillon liquide **2** se trouve absorbé complétement par le support hydratant **3**. Ainsi, en fonction du nombre de puits **6** recevant un échantillon liquide, les puits sont dimensionnés en volume pour obtenir l'absorption complète de l'échantillon liquide par le support hydratant **3.** A cet effet, chaque puits **6** possède une profondeur limitée, typiquement comprise entre 1 et 5 mm, encore plus préférentiellement entre 2 et 3 mm. Par ailleurs, selon une caractéristique avantageuse, le volume calibré des puits de réception **6** incluant éventuellement les micro-canaux **16** est déterminé de sorte que chaque support hydratant **3** se trouve complètement hydraté par un échantillon liquide **2.**

Selon une caractéristique avantageuse de réalisation de l'invention, les supports hydratants **3** sont espacés les uns des autres par des couloirs de séparation **18** de manière à séparer les supports hydratants entre eux et par suite, les échantillons liquides. Une telle disposition interdit ainsi une contamination croisée entre les supports hydratants **3.** Typiquement, les supports hydratants **3** sont séparés les uns des autres par une distance de l'ordre de 3 mm. De plus, l'aménagement de couloirs **18** entre les supports hydratants **3** assure une circulation de l'air entre les supports hydratants **3** et une bonne aération notamment lors de la phase d'incubation comme cela sera expliqué dans la suite de la description.

Selon un mode préféré de réalisation, lorsque le couvercle **5** est pourvu de plusieurs supports hydratants **3** destinés à être hydratés chacun par un échantillon liquide, le nombre et le volume des puits **6** en regard de chaque support hydratant sont identiques pour tous les supports hydratants. Un tel aménagement permet d'assurer une comparaison en ce qui concerne la réaction du milieu réactionnel. Bien entendu, il peut être envisagé un aménagement différent dans lequel le nombre et le volume des puits **6** en regard de chaque support hydratant sont différents pour les supports hydratants.

Bien entendu, le réceptacle **4** peut comporter au moins une cloison de séparation en vue de délimiter au moins deux cuvettes **13** pour répartir dans chacune d'entre elles, un échantillon liquide différent d'une cuvette à l'autre. Cette cloison de séparation est aménagée pour relier deux zones de la bordure périphérique **14** situées selon deux côtés opposés ou adjacents du réceptacle. Les cuvettes **13** peuvent ainsi recevoir des échantillons liquides différents en termes de composition et/ou de dilution. De même, une cuvette **13** peut être destinée à recevoir un échantillon liquide ayant comme finalité d'hydrater un support hydratant pour maintenir un taux d'humidité déterminé à l'intérieur du dispositif **1.**

Selon les variantes de réalisation décrites ci-avant, chaque cuvette **13** est aménagée pour confiner le ou les échantillons liquides dans la mesure où la plaque **9** et la bordure périphérique **14** ne comporte pas de trous. Selon une autre variante de réalisation, il est à noter que la cuvette **13** peut comporter au moins un orifice de sortie pour permettre d'évacuer l'excédent de l'échantillon liquide versé. Dans ce cas, on verse un échantillon liquide d'un volume supérieur au volume total des puits et l'excédent est vidé par un puits percé positionné à l'extérieur des supports hydratants. Une telle variante de réalisation trouve par exemple une application avantageuse pour un support hydratant de détection du Choléra.

Selon une caractéristique avantageuse de réalisation du dispositif **1** selon l'invention, le couvercle **5** possède une rainure périphérique **19** apte à venir s'emboîter dans la bordure périphérique **17** du réceptacle en position d'hydratation.

Avantageusement, le dispositif **1** selon l'invention comporte un système **23** de centrage relatif entre le couvercle **5** et le réceptacle **4** pour positionner chaque support hydratant **3** en position superposée par rapport à un ou plusieurs puits de réception d'un échantillon liquide. Selon une caractéristique préférée de réalisation, ce système de centrage est réalisé par la rainure périphérique **19** s'engageant dans la bordure périphérique **14** du réceptacle. Ainsi, l'assemblage entre le couvercle **5** et le réceptacle **4** permet d'obtenir un positionnement précis des supports hydratants **3** qui viennent recouvrir complètement chacun, l'orifice du ou des puits contenant un échantillon liquide.

Selon une variante de réalisation, la rainure périphérique **19** et la bordure périphérique **14** sont ajustées de manière à obtenir un emboîtement étanche.

Selon une autre variante de réalisation, la rainure périphérique **19** et la bordure périphérique **14** sont aménagées pour permettre une circulation de l'air entre le couvercle et le réceptacle afin d'obtenir une ventilation contrôlée des supports hydratants. Par exemple et comme cela apparaît à la **Fig. 16****,** la rainure périphérique **19** est pourvue localement de butées **19₁** venant en appui sur la bordure périphérique **14** du réceptacle autorisant la circulation de l'air entre ces butées.

Il doit être compris que l'engagement du couvercle **5** sur le réceptacle **4** par la coopération de la rainure périphérique **19** dans la bordure périphérique **14** permet un guidage et un rapprochement relatif de manière à amener chaque support hydratant **3,** à venir en contact avec l'échantillon liquide. A cet effet, chaque support hydratant **3** est destiné à venir en contact ou en butée contre les rebords périphériques **12** des puits de sorte que la face d'absorption **3₁** des supports hydratants est positionnée au niveau des bords supérieurs **8** des puits. Dans cette position dite d'hydratation, l'échantillon liquide se trouve absorbé par capillarité par le support hydratant placé en vis-à-vis.

Il est à noter que le dispositif **1** selon l'invention peut comporter un système **24** de verrouillage du couvercle **5** et du réceptacle **4** dans une position stable correspondant à la position d'hydratation de manière à maintenir en position fermée le couvercle et le réceptacle. Un tel système **24** de verrouillage est par exemple réalisé par un ergot aménagé sur la bordure périphérique **14** du réceptacle pour bloquer en position fermée, le couvercle **5** sur le réceptacle **4.**

Selon une caractéristique avantageuse de réalisation, le réceptacle **4** et/ou le couvercle **5** sont réalisés en un matériau transparent ou translucide pour permettre une lecture directe de l'hydratation ou de l'activation réalisée. Par exemple, le réceptacle **4** et le couvercle **5** sont réalisés en PET polyéthylène téréphtalate ou autre exemple comme le PVC polychlorure de vinyl ou encore en PP polypropylène ou en PLA polylactique bioplastique d'origine végétale renouvelable et biodégradable, par les techniques classiques de thermoformage ou de moulage par injection. De manière complémentaire, le réceptacle **4** et/ou le couvercle **5** peuvent intégrer des systèmes optiques permettant d'améliorer la qualité visuelle des supports hydratants **3.** De plus, le réceptacle **4** et le couvercle **5** possèdent des formes complémentaires, à savoir rectangulaire dans les exemples illustrés mais il est clair que la forme du réceptacle et du couvercle peut être différente. Enfin, le réceptacle **4** et le couvercle **5** peuvent être articulés ensemble autour de charnières ou se présenter sous la forme de 2 pièces indépendantes.

Le dispositif **1** selon l'invention permet la mise en œuvre simple, d'un procédé pour l'hydratation de supports hydratants par des échantillons liquides.

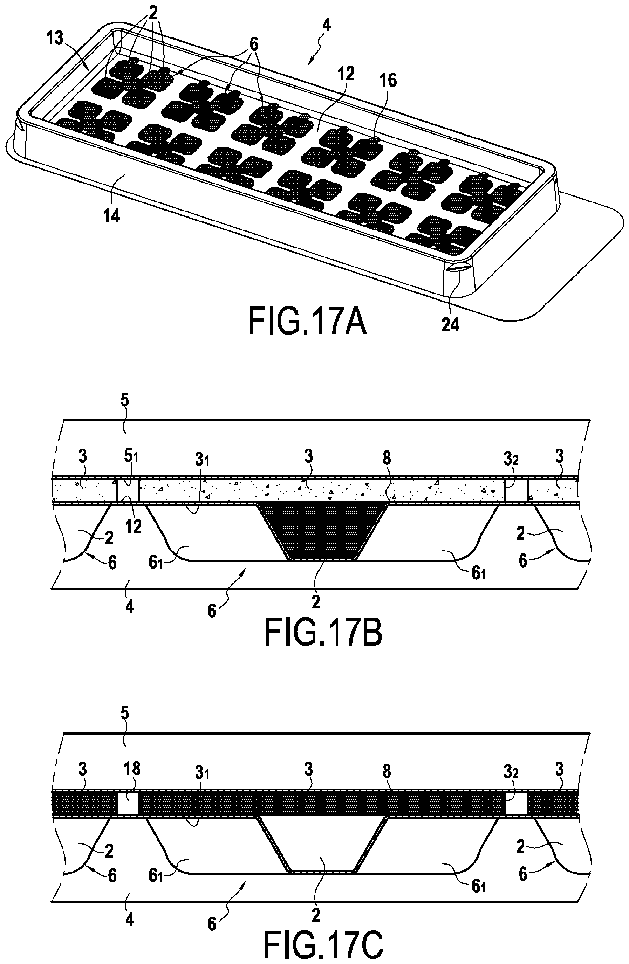

La première étape consiste à répartir complètement chaque échantillon liquide dans un ou plusieurs puits **6** du réceptacle **4.** Dans l'exemple illustré à la **Fig. 17A****,** chaque échantillon est à répartir dans un puits **6** comportant quatre cavités reliées entre elles conformément à la **Fig. 13****.** Bien entendu, dans le cas où le couvercle **5** est positionné sur le réceptacle, le couvercle **5** est enlevé pour permettre de verser le volume d'échantillon à l'intérieur de la cuvette **13** de répartition du liquide. Dans un exemple considéré, chaque échantillon liquide **2** présente un volume de 500 microlitres destiné à être réparti dans les quatre cuvettes du puits **6** reliées entre elles. Le couvercle **5** possède douze supports hydratants **3** de sorte que le réceptacle **4** est destiné à recevoir douze volumes d'échantillons liquides à savoir un volume total de 6 millilitres. Un volume de liquide de 6 millilitres est donc versé dans la cuvette **13** du réceptacle **3.** Cette répartition est définie par le volume présenté par les puits **6.**

La répartition de chaque échantillon liquide est effectuée par légère inclinaison et/ou agitation du réceptacle **4** afin de pouvoir répartir le liquide dans les différents puits. Cette agitation est manuelle ou peut être réalisée éventuellement par un mécanisme automatisé. Cette agitation est arrêtée lorsque tous les puits **6** sont remplis par l'échantillon liquide c'est-à-dire lorsque le niveau du liquide atteint le bord supérieur **8** des puits.

Au terme de cette étape, il est à noter qu'entre chaque puits, il n'y a pas de liquide résiduel, ce qui permet d'avoir une séparation physique entre chaque puits et donc d'éviter un risque de contamination croisée entre les supports hydratants **3.** Le liquide est maintenu dans les puits grâce à la forme et aux géométries particulières qui favorisent la rétention du liquide dans les puits et par les propriétés hydrophiles du matériau constitutif du réceptacle **4.** Cette opération de répartition des échantillons liquides est une opération simple, facile à mener à bien, précise et sans risque de débordement car le liquide reste confiné à l'intérieur de la cuvette **16** à l'aide de la bordure périphérique **14.**

L'étape suivante consiste à positionner le couvercle **5** et le réceptacle **4** dans une position d'hydratation dans laquelle chaque support hydratant **3** est en contact avec un échantillon liquide. A cet effet, le couvercle **5** et le réceptacle **4** sont rapprochés de manière que les supports hydratants **3** viennent en position superposée puis en contact avec les rebords périphériques **12** des puits **7.** Dans cette position de butée illustrée à la **Fig. 17B****,** les supports hydratants **3** sont en contact chacun par leurs faces d'absorption **3₁**, avec un échantillon liquide remplissant le ou les puits, permettant ainsi l'absorption par capillarité, de chaque échantillon liquide par son support hydratant placé en regard. Chaque échantillon liquide **2** contenu dans le puits **6** est transféré par capillarité instantanément et simultanément pour tous les supports hydratants **3** (**Fig. 17C**). Le transfert de l'échantillon liquide dans le support hydratant **3** est réalisé sans l'apparition d'un front de migration du liquide. L'homogénéité du milieu réactionnel imprégnant le support hydratant **3** n'est donc pas modifiée. Les supports hydratants **3** étant séparés physiquement, le risque de contamination croisée est très limité.

Le couvercle **5** et le réceptacle **4** sont maintenus dans cette position d'hydratation pour permettre la réalisation d'une étape d'incubation. Les échantillons liquides **2** étant piégés dans les supports hydratants **3,** l'incubation peut être réalisée dans n'importe quelle position du dispositif sans risque de voir couler l'échantillon liquide. Il est à noter que les couloirs **18** aménagés entre les supports hydratants **3** autorisent le passage d'air pour favoriser la croissance lors de l'incubation. De même, les puits vides **6** laissent un volume d'air disponible pour favoriser la croissance lors de l'incubation. Les micro-canaux **16** d'alimentation une fois vidée permettent de faire circuler l'air dans la cavité vidée de l'échantillon. Le réceptacle **4** et couvercle **5** sont ajustés de manière à contrôler, en fonction des applications visées, le passage d'air à l'intérieur du dispositif. Il peut ainsi être prévu de réaliser soit une étanchéité permettant de limiter l'évaporation lors de l'incubation et le dessèchement des supports hydratants, soit une ventilation contrôlée des supports hydratants pour favoriser par exemple la croissance microbienne lors de l'incubation.

Au terme de l'étape d'incubation, une étape de lecture peut être réalisée pour lire chaque support hydratant **3** afin de détecter et/ou identifier et/ou énumérer au moins un microorganisme cible dans un échantillon liquide susceptible de le contenir. Avantageusement, cette étape de lecture de chaque support hydratant **3** est réalisée directement à travers le réceptacle et/ou le couvercle, à l'œil nu ou à l'aide de systèmes d'imagerie ou à l'aide d'un accessoire de lecture. Selon une autre variante de réalisation, il est à noter qu'après l'étape d'incubation, le réceptacle **4** peut être remplacé par un fond plan transparent ou translucide à travers lequel, l'opération de lecture de chaque support hydratant est réalisée.

## Revendications

1. Dispositif pour hydrater par un échantillon liquide (**2**), un support hydratant **(3)** comprenant un milieu réactionnel comprenant tous les éléments nécessaires à la survie et/ou à la croissance des microorganismes, le dispositif comportant :
- un réceptacle **(4)** de réception d'au moins un échantillon liquide **(2),** ce réceptacle comprenant au moins un puits **(6)** de réception ouvert vers le haut au niveau d'un bord supérieur **(8)** ;
- un couvercle **(5)** possédant une face inférieure (**5₁**) sur laquelle au moins un support hydratant **(3)** est fixé pour présenter une face d'absorption (**3₁**) pour un échantillon liquide lorsque le couvercle et le réceptacle occupent une position d'hydratation dans laquelle le support hydratant **(3)** est en contact avec l'échantillon liquide selon une interface d'hydratation ;
**caractérisé en ce que** le réceptacle **(4)** présente, par au moins un puits **(6),** un volume calibré de réception d'un échantillon liquide, l'au moins dit puits présentant une section ouverte calibrée d'hydratation d'un support hydratant **(3)** délimitée par le bord supérieur **(8)** d'au moins ledit puits **(6),** cette section ouverte calibrée d'hydratation possédant une surface inférieure à la surface de la face d'absorption du support hydratant **(3)** pour assurer le contrôle de l'absorption par capillarité, de l'échantillon liquide par le support hydratant.

2. Dispositif selon la revendication 1, **caractérisé en ce que** la surface de la section ouverte calibrée d'hydratation du support hydratant par un échantillon liquide **(2)** délimitée par au moins un puits, est telle que le ratio de cette surface sur la surface de la face d'absorption (**3₁**) du support hydratant **(3)** est compris entre 40 et 80 % et de préférence entre 55 et 65 %.

3. Dispositif selon les revendications 1 ou 2, **caractérisé en ce que** le réceptacle **(4)** comporte des puits de réception **(6)** pour plusieurs échantillons liquides et **en ce que** le couvercle **(5)** est équipé de plusieurs supports hydratants **(3)** espacés les uns des autres par des couloirs de séparation et destinés, en position d'hydratation, à être hydratés chacun par un échantillon liquide (**2**).

4. Dispositif selon l'une des revendications 1 à 3, **caractérisé en ce que** la surface de la section ouverte calibrée d'hydratation d'un support hydratant **(3)** par un échantillon liquide est délimitée par plusieurs puits **(6)** présentant chacun une section ouverte calibrée d'hydratation et qui, en combinaison avec les autres sections des puits, présentent au total, une surface correspondant à la surface de la section ouverte calibrée d'hydratation pour un support hydratant.

5. Dispositif selon l'une des revendications 1 à 3, **caractérisé en ce que** la surface de la section ouverte calibrée d'hydratation d'un support hydratant par un échantillon liquide **(2)** est délimitée par un unique puits **(6).**

6. Dispositif selon l'une des revendications 1 à 5, **caractérisé en ce que** chaque puits **(6)** comporte plusieurs cavités (**6₁**) de réception pour une partie de l'échantillon liquide, ces cavités de réception communiquant entre-elles au moins au niveau de la section ouverte calibrée d'hydratation dont le contour possède différents lobes.

7. Dispositif selon l'une des revendications 1 à 6, **caractérisé en ce que** le réceptacle **(4)** comporte au moins un puits **(6)** communiquant avec un micro-canal (**16**) d'alimentation dont une partie est située en dehors de l'interface d'hydratation, le puits **(6)** et le micro-canal **(16)** délimitant un volume calibré de réception pour un échantillon liquide et présentant une section ouverte calibrée d'hydratation délimitée par le bord supérieur **(8)** du puits **(6)** et au moins une partie du bord supérieur du micro-canal **(16).**

8. Dispositif selon la revendication 7, **caractérisé en ce que** chaque puits **(6)** présente une section ouverte calibrée d'hydratation délimitée par un contour qui en dehors des micro-canaux **(16),** laisse subsister, en position d'hydratation, sur la face d'absorption du support hydratant, une ceinture périphérique entre ledit contour et le bord périphérique du support hydratant.

9. Dispositif selon l'une des revendications 1 à 8, **caractérisé en ce que** chaque puits **(6)** présente une section ouverte calibrée d'hydratation délimitée par un contour dont la forme est choisie de manière qu'aucun des points pris à l'intérieur de ladite section, n'est situé à une distance linéaire supérieure de 4 mm par rapport au contour le plus proche de ladite section.

10. Dispositif selon l'une des revendications 1 à 9, **caractérisé en ce que** chaque puits **(6)** présente une section ouverte calibrée d'hydratation délimitée par un contour dont la forme est choisie de manière que la plus petite dimension de cette section soit supérieure à 1,5 mm.

11. Dispositif selon l'une des revendications 1 à 10, **caractérisé en ce que** chaque puits **(6)** présente une section ouverte calibrée d'hydratation délimitée par un contour dont la forme est choisie de manière qu'aucun des points pris entre l'extérieur de ladite section et le contour de la face d'absorption (**3₁**) du support hydratant, n'est situé à une distance linéaire supérieure de 3 mm par rapport aux contours les plus proches de ladite face ou de ladite section.

12. Dispositif selon l'une des revendications 1 à 11, **caractérisé en ce que** le couvercle **(5)** est pourvu de plusieurs supports hydratants **(3)** destinés à être hydratés chacun par un échantillon liquide et **en ce que** le nombre et le volume des puits **(6)** en regard de chaque support hydratant sont identiques pour tous les supports hydratants.

13. Dispositif selon l'une des revendications 1 à 12, **caractérisé en ce que** le ou les puits **(6)** incluant les mini-canaux de réception d'un échantillon liquide **(2)** délimitent un volume calibré adapté afin qu'en position d'hydratation du couvercle **(4)** et du réceptacle (**5**), l'échantillon liquide se trouve absorbé complètement par le support hydratant **(3).**

14. Dispositif selon l'une des revendications 1 à 13, **caractérisé en ce que** le ou les puits **(6)** incluant les micro-canaux **(16)** de réception d'un échantillon liquide **(2)** délimitent un volume calibré adapté afin qu'en position d'hydratation du couvercle **(4)** et du réceptacle (**5**), le support hydratant **(3)** se trouve complètement hydraté.

15. Dispositif selon l'une des revendications 1 à 14, **caractérisé en ce qu'**au moins un puits **(6)** présente un profil qui se rétrécit à partir de son bord supérieur **(8).**

16. Dispositif selon l'une des revendications 1 à 15, **caractérisé en ce que** le réceptacle **(4)** comporte une plaque **(9)** dans laquelle le ou les puits et les micro-canaux sont aménagés en étant entourés par un rebord périphérique **(12)** servant, en position d'hydratation, de surface de butée pour les supports hydratants.

17. Dispositif selon l'une des revendications 1 à 16, **caractérisé en ce que** le réceptacle **(4)** comporte une plaque **(9)** à partir de laquelle s'élève en saillie, une bordure périphérique **(14)** entourant les puits et délimitant intérieurement au moins une cuvette **(13)** de répartition pour le ou les échantillons liquides.

18. Dispositif selon la revendication 17, **caractérisé en ce que** le couvercle **(5)** possède une rainure périphérique **(19)** apte à s'emboîter dans la bordure périphérique **(14)** du réceptacle **(4)** pour assurer un emboîtement étanche ou avec une ventilation contrôlée.

19. Dispositif selon l'une des revendications 1 à 18, **caractérisé en ce qu'**il comporte un système **(23)** de centrage relatif entre le couvercle **(5)** et le réceptacle **(4)** pour positionner chaque support hydratant **(3)** en position superposée par rapport à un ou plusieurs puits **(6)** de réception d'un échantillon liquide (**2**).

20. Dispositif selon l'une des revendications 1 à 19, **caractérisé en ce qu'**il comporte un système **(24)** de verrouillage du couvercle **(5)** et du réceptacle **(4)** dans une position stable fermée correspondant à la position d'hydratation.

21. Dispositif selon l'une des revendications 1 à 20, **caractérisé en ce que** le réceptacle **(4)** et/ou le couvercle **(5)** sont réalisés en un matériau transparent ou translucide.

22. Dispositif selon l'une des revendications 1 à 21, **caractérisé en ce que** chaque puits **(6)** est pourvu d'un colorant pour l'échantillon liquide afin d'aider au remplissage dudit puits.

23. Utilisation d'un dispositif **(1)** selon l'une des revendications 1 à 22, pour détecter et/ou identifier et/ou énumérer au moins un microorganisme dans un échantillon liquide **(2)** susceptible de le contenir.

24. Utilisation d'un dispositif **(1)** selon l'une des revendications 1 à 22, pour déterminer la sensibilité et la résistance d'au moins un microorganisme à au moins un antibiotique.

25. Procédé pour hydrater au moins un support hydratant par un échantillon liquide (**2**), à l'aide d'un dispositif **(1)** conforme à l'une des revendications 1 à 22, afin de détecter et/ou identifier et/ou énumérer au moins un microorganisme cible dans un échantillon liquide susceptible de le contenir, **caractérisé en ce qu'**il comporte les étapes suivantes :
- répartir complètement chaque échantillon liquide **(2)** dans un ou plusieurs puits **(6)** ;
- positionner le couvercle **(5)** et le réceptacle **(4)** dans leur position d'hydratation dans laquelle chaque support hydratant **(3)** est en contact avec un échantillon liquide **(2)** ;
- maintenir le couvercle **(5)** et le réceptacle **(4)** en position d'hydratation pour la réalisation d'une étape d'incubation ;
- et lire chaque support hydratant **(3)** afin de détecter et/ou identifier et/ou énumérer au moins un microorganisme cible.

26. Procédé selon la revendication 25, **caractérisé en ce qu'**il consiste à répartir chaque échantillon liquide **(2)** en versant dans le réceptacle **(4)** le ou les échantillons liquides et en agitant le réceptacle **(4)** afin de s'assurer que chaque échantillon liquide **(2)** remplisse complètement le ou les puits de réception **(6).**

27. Procédé selon l'une des revendications 25 ou 26, **caractérisé en ce qu'**il consiste à réaliser l'étape de lecture de chaque support hydratant **(3)** à travers le réceptacle **(4)** et/ou le couvercle **(5).**

28. Procédé selon l'une des revendications 25 à 27, **caractérisé en ce qu'**il consiste après l'étape d'incubation, à remplacer le réceptacle **(4)** par un fond plan transparent ou translucide à travers lequel l'opération de lecture de chaque support hydratant **(3)** est réalisée.

## Patentansprüche

1. Vorrichtung zum Hydratisieren, mittels einer flüssigen Probe (2), eines hydratisierenden Trägers (3), der ein Reaktionsmedium umfasst, welches alle für das Überleben und/oder für das Wachstum der Mikroorganismen erforderlichen Elemente enthält, wobei die Vorrichtung umfasst:
- einen Behälter (4) zur Aufnahme wenigstens einer flüssigen Probe (2), wobei dieser Behälter wenigstens eine Aufnahmevertiefung (6) aufweist, die im Bereich eines oberen Randes (8) nach oben hin offen ist,
- einen Deckel (5), der eine Unterseite (5₁) besitzt, auf der wenigstens ein hydratisierender Träger (3) befestigt ist, um eine Absorptionsseite(3₁) für eine flüssige Probe aufzuweisen, wenn der Deckel und der Behälter eine Hydratisierungsposition einnehmen, in welcher der hydratisierende Träger (3) entlang einer Hydratisierungsgrenzfläche mit der flüssigen Probe in Kontakt ist,
**dadurch gekennzeichnet, dass** der Behälter (4) durch wenigstens eine Vertiefung (6) ein kalibriertes Volumen zur Aufnahme einer flüssigen Probe aufweist, wobei die wenigstens eine Vertiefung einen kalibrierten offenen Querschnitt zum Hydratisieren eines hydratisierenden Trägers (3) aufweist, der durch den oberen Rand (8) von wenigstens der Vertiefung (6) begrenzt ist, wobei dieser kalibrierte offene Hydratisierungsquerschnitt eine Fläche aufweist, die kleiner als die Absorptionsseite des hydratisierenden Trägers (3) ist, um die Kontrolle der Kapillareffekt-Absorption der flüssigen Probe durch den hydratisierenden Träger sicherzustellen.

2. Vorrichtung nach Anspruch 1, **dadurch gekennzeichnet, dass** die Fläche des durch wenigstens eine Vertiefung begrenzten kalibrierten offenen Querschnitts zum Hydratisieren des hydratisierenden Trägers mittels einer flüssigen Probe (2) derart ist, dass das Verhältnis dieser Fläche zu der Fläche der Absorptionsseite (3₁) des hydratisierenden Trägers (3) zwischen 40 und 80 % und vorzugsweise zwischen 55 und 65 % beträgt.

3. Vorrichtung nach den Ansprüchen 1 oder 2, **dadurch gekennzeichnet, dass** der Behälter (4) Aufnahmevertiefungen (6) für mehrere flüssige Proben aufweist und dass der Deckel (5) mit mehreren hydratisierenden Trägern (3) ausgestattet ist, die durch Trenngänge voneinander beabstandet und in der Hydratisierungsposition dazu bestimmt sind, jeweils mittels einer flüssigen Probe (2) hydratisiert zu werden.

4. Vorrichtung nach einem der Ansprüche 1 bis 3, **dadurch gekennzeichnet, dass** die Fläche des kalibrierten offenen Querschnitts zum Hydratisieren eines hydratisierenden Trägers (3) mittels einer flüssigen Probe durch mehrere Vertiefungen (6) begrenzt ist, die jeweils einen kalibrierten offenen Hydratisierungsquerschnitt aufweisen und die in Kombination mit den anderen Querschnitten der Vertiefungen insgesamt eine Fläche aufweisen, welche der Fläche des kalibrierten offenen Hydratisierungsquerschnitts für einen hydratisierenden Träger entspricht.

5. Vorrichtung nach einem der Ansprüche 1 bis 3, **dadurch gekennzeichnet, dass** die Fläche des kalibrierten offenen Querschnitts zum Hydratisieren eines hydratisierenden Trägers mittels einer flüssigen Probe (2) durch eine einzige Vertiefung (6) begrenzt ist.

6. Vorrichtung nach einem der Ansprüche 1 bis 5, **dadurch gekennzeichnet, dass** jede Vertiefung (6) mehrere Aufnahmehohlräume (6₁) für einen Teil der flüssigen Probe aufweist, wobei diese Aufnahmehohlräume wenigstens im Bereich des kalibrierten offenen Hydratisierungsquerschnitts, dessen Kontur verschiedene Lappen aufweist, miteinander in Verbindung stehen.

7. Vorrichtung nach einem der Ansprüche 1 bis 6, **dadurch gekennzeichnet, dass** der Behälter (4) wenigstens eine Vertiefung (6) aufweist, die mit einem Versorgungsmikrokanal (16) in Verbindung steht, von welchem ein Teil außerhalb der Hydratisierungsgrenzfläche gelegen ist, wobei die Vertiefung (6) und der Mikrokanal (16) ein kalibriertes Aufnahmevolumen für eine flüssige Probe begrenzen und einen kalibrierten offenen Hydratisierungsquerschnitt aufweisen, der durch den oberen Rand (8) der Vertiefung (6) und wenigstens einen Teil des oberen Randes des Mikrokanals (16) begrenzt ist.

8. Vorrichtung nach Anspruch 7, **dadurch gekennzeichnet, dass** jede Vertiefung (6) einen kalibrierten offenen Hydratisierungsquerschnitt aufweist, der durch eine Kontur begrenzt ist, die außerhalb der Mikrokanäle (16) in der Hydratisierungsposition auf der Absorptionsseite des hydratisierenden Trägers einen umlaufenden Gürtel zwischen der Kontur und dem Umfangsrand des hydratisierenden Trägers bestehen lässt.

9. Vorrichtung nach einem der Ansprüche 1 bis 8, **dadurch gekennzeichnet, dass** jede Vertiefung (6) einen kalibrierten offenen Hydratisierungsquerschnitt aufweist, der durch eine Kontur begrenzt ist, deren Form derart gewählt ist, dass keiner der Punkte innerhalb des Querschnitts in einem linearen Abstand von mehr als 4 mm von der am nächsten befindlichen Kontur des Querschnitts gelegen ist.

10. Vorrichtung nach einem der Ansprüche 1 bis 9, **dadurch gekennzeichnet, dass** jede Vertiefung (6) einen kalibrierten offenen Hydratisierungsquerschnitt aufweist, der durch eine Kontur begrenzt ist, deren Form derart gewählt ist, dass die kleinste Abmessung dieses Querschnitts größer als 1,5 mm ist.

11. Vorrichtung nach einem der Ansprüche 1 bis 10, **dadurch gekennzeichnet, dass** jede Vertiefung (6) einen kalibrierten offenen Hydratisierungsquerschnitt aufweist, der durch eine Kontur begrenzt ist, deren Form derart gewählt ist, dass keiner der Punkte zwischen der Außenseite des Querschnitts und der Kontur der Absorptionsseite (3₁) des hydratisierenden Trägers in einem linearen Abstand von mehr als 3 mm von den am nächsten befindlichen Konturen der Seite oder des Querschnitts gelegen ist.

12. Vorrichtung nach einem der Ansprüche 1 bis 11, **dadurch gekennzeichnet, dass** der Deckel (5) mit mehreren hydratisierenden Trägern (3) versehen ist, die dazu bestimmt sind, jeweils mittels einer flüssigen Probe hydratisiert zu werden, und dass die Anzahl und das Volumen der Vertiefungen (6) gegenüber einem jeden hydratisierenden Träger für alle hydratisierenden Träger identisch sind.

13. Vorrichtung nach einem der Ansprüche 1 bis 12, **dadurch gekennzeichnet, dass** der oder die Vertiefung(en) (6), welche die Minikanäle zur Aufnahme einer flüssigen Probe (2) einschließen, ein geeignetes kalibriertes Volumen begrenzen, damit in der Hydratisierungsposition des Deckels (4) und des Behälters (5) die flüssige Probe vollständig durch den hydratisierenden Träger (3) aufgenommen ist.

14. Vorrichtung nach einem der Ansprüche 1 bis 13, **dadurch gekennzeichnet, dass** der oder die Vertiefung(en) (6), welche die Mikrokanäle zur Aufnahme einer flüssigen Probe (2) einschließen, ein geeignetes kalibriertes Volumen begrenzen, damit in der Hydratisierungsposition des Deckels (4) und des Behälters (5) der hydratisierende Träger (3) vollständig hydratisiert ist.

15. Vorrichtung nach einem der Ansprüche 1 bis 14, **dadurch gekennzeichnet, dass** wenigstens eine Vertiefung (6) ein Profil aufweist, das sich von ihrem oberen Rand (8) ausgehend verjüngt.

16. Vorrichtung nach einem der Ansprüche 1 bis 15, **dadurch gekennzeichnet, dass** der Behälter (4) eine Platte (9) umfasst, in der die Vertiefung oder Vertiefungen und die Mikrokanäle von einem umlaufenden Rand (12) umgeben angeordnet sind, welcher in der Hydratisierungsposition als Anschlagfläche für die hydratisierenden Träger dient.

17. Vorrichtung nach einem der Ansprüche 1 bis 16, **dadurch gekennzeichnet, dass** der Behälter (4) eine Platte (9) umfasst, von der ausgehend sich ein umlaufender Rand (14) erhebt, welcher die Vertiefungen umgibt und innen wenigstens eine Verteilungsschale (13) für die flüssige Probe oder flüssigen Proben begrenzt.

18. Vorrichtung nach Anspruch 17, **dadurch gekennzeichnet, dass** der Deckel (5) eine umlaufende Nut (19) aufweist, die geeignet ist, auf den umlaufenden Rand (14) des Behälters (4) aufgesteckt zu werden, um ein dichtes Ineinandergreifen oder Ineinandergreifen mit einer kontrollierten Belüftung sicherzustellen.

19. Vorrichtung nach einem der Ansprüche 1 bis 18, **dadurch gekennzeichnet, dass** sie ein System (23) zum relativen Zentrieren zwischen dem Deckel (5) und dem Behälter (4) umfasst, um jeden hydratisierenden Träger (3) in überlagerter Position bezüglich einer oder mehrerer Vertiefung(en) (6) zur Aufnahme einer flüssigen Probe (2) zu positionieren.

20. Vorrichtung nach einem der Ansprüche 1 bis 19, **dadurch gekennzeichnet, dass** sie ein System (24) zum Verriegeln des Deckels (5) und des Behälters (4) in einer stabilen geschlossenen Position, die der Hydratisierungsposition entspricht, umfasst.

21. Vorrichtung nach einem der Ansprüche 1 bis 20, **dadurch gekennzeichnet, dass** der Behälter (4) und/oder der Deckel (5) aus einem transparenten oder transluzenten Material gefertigt sind.

22. Vorrichtung nach einem der Ansprüche 1 bis 21, **dadurch gekennzeichnet, dass** jede Vertiefung (6) mit einem Farbstoff für die flüssige Probe versehen ist, um beim Befüllen der Vertiefung zu helfen.

23. Verwendung einer Vorrichtung (1) nach einem der Ansprüche 1 bis 22 zum Nachweis und/oder zur Erkennung und/oder zum Aufzählen wenigstens eines Mikroorganismus in einer flüssigen Probe (2), die geeignet ist, ihn zu enthalten.

24. Verwendung einer Vorrichtung (1) nach einem der Ansprüche 1 bis 22 zur Bestimmung der Empfindlichkeit und der Widerstandsfähigkeit wenigstens eines Mikroorganismus gegenüber wenigstens einem Antibiotikum.

25. Verfahren zum Hydratisieren wenigstens eines hydratisierenden Trägers mittels einer flüssigen Probe (2) mit Hilfe einer Vorrichtung (1) nach einem der Ansprüche 1 bis 22, um wenigstens einen Zielmikroorganismus in einer flüssigen Probe, welche geeignet ist, ihn zu enthalten, nachzuweisen und/oder zu erkennen und/oder aufzuzählen, **dadurch gekennzeichnet, dass** es die folgenden Schritte umfasst:
- vollständiges Verteilen jeder flüssigen Probe (2) in eine oder mehrere Vertiefung(en) (6),
- Positionieren des Deckels (5) und des Behälters (4) in ihrer Hydratisierungsposition, in der jeder hydratisierende Träger (3) mit einer flüssigen Probe (2) in Kontakt ist,
- Halten des Deckels (5) und des Behälters (4) in der Hydratisierungsposition für die Durchführung eines Inkubationsschrittes,
- und Auslesen eines jeden hydratisierenden Trägers (3), um wenigstens einen Zielmikroorganismus nachzuweisen und/oder zu erkennen und/oder aufzuzählen.

26. Verfahren nach Anspruch 25, **dadurch gekennzeichnet, dass** es darin besteht, jede flüssige Probe (2) dadurch zu verteilen, dass die flüssige Probe oder flüssigen Proben in den Behälter (4) gegossen wird/werden und dass der Behälter (4) geschüttelt wird, um sicher zu gehen, dass jede flüssige Probe (2) die Aufnahmevertiefung oder -vertiefungen (6) vollständig ausfüllt.

27. Verfahren nach einem der Ansprüche 25 oder 26, **dadurch gekennzeichnet, dass** es darin besteht, den Schritt des Auslesens eines jeden hydratisierenden Trägers (3) durch den Behälter (4) und/oder den Deckel (5) hindurch durchzuführen.

28. Verfahren nach einem der Ansprüche 25 bis 27, **dadurch gekennzeichnet, dass** es nach dem Inkubationsschritt darin besteht, den Behälter (4) durch einen transparenten oder transluzenten planen Boden zu ersetzen, durch den hindurch der Vorgang des Auslesens eines jeden hydratisierenden Trägers (3) erfolgt.

## Claims

1. A device for hydrating a hydrating support (3) that contains a reaction medium including all of the elements necessary for the survival and / or growth of microorganisms with a liquid sample (2), the device comprising:
- a container (4) for receiving at least one liquid sample (2), the container including at least one reception well (6) that is upwardly open at a top edge (8); and
- a lid (5) possessing a bottom face (5₁) having at least one hydrating support (3) fastened thereto to present an absorption face (3₁) for absorbing a liquid sample when the lid and the container occupy a hydrating position in which the hydrating support (3) is in contact with the liquid sample via a hydrating interface;
the device being **characterized in that** the container (4) presents, by means of at least one well (6), a calibrated volume for receiving a liquid sample, the at least said well presenting a hydrating calibrated open section for hydrating a hydrating support (3) defined by the top edge (8) of at least said well (6), the hydrating calibrated open section possessing an area that is less than the area of the absorption face of the hydrating support (3) in order to control the absorption by capillarity of the liquid sample by the hydrating support.

2. A device according to claim 1, **characterized in that** the area of the calibrated open section for hydrating a hydrating support by a liquid sample (2) defined by at least one well is such that the ratio of this area over the area of the absorption face (3₁) of the hydrating support (3) lies in the range 40% to 80% and preferably in the range 55% to 65%.

3. A device according to claim 1 or claim 2, **characterized in that** the container (4) includes reception wells (6) for a plurality of liquid samples, and **in that** the lid (5) is fitted with a plurality of hydrating supports (3) that are spaced apart from one another by separation corridors and each of which is to be hydrated, in the hydrating position by a respective liquid sample (2).

4. A device according to any one of claims 1 to 3, **characterized in that** the area of the calibrated open section for hydrating a hydrating support (3) by a liquid sample is defined by a plurality of wells (6), each presenting a hydrating calibrated open section, which section in combination with the other well sections, present a total area corresponding to the area of the calibrated open section for hydrating a hydrating support.

5. A device according to any one of claims 1 to 3, **characterized in that** the area of the calibrated open section for hydrating a hydrating support with a liquid sample (2) is defined by a single well (6).

6. A device according to any one of claims 1 to 5, **characterized in that** each well (6) includes a plurality of reception cavities (6₁) for respective portions of the liquid sample, the reception cavities communicating with one another at least via the hydrating calibrated open section, which is of outline that possesses various lobes.

7. A device according to any one of claims 1 to 6, **characterized in that** the container (4) includes at least one well (6) communicating with a feed microchannel (16) having a portion situated outside the hydrating interface, the well (6) and the microchannel (16) defining a calibrated reception volume for receiving a liquid sample and presenting a hydrating calibrated open section defined by the top edge (8) of the well (6) and at least a portion of the top edge of the microchannel (16).

8. A device according to claim 7, **characterized in that** each well (6) presents a hydrating calibrated open section defined by an outline that is outside the microchannels (16), thereby leaving a peripheral belt in the hydrating position on the absorption face of the hydrating support, which belt lies between said outline and the peripheral edge of the hydrating support.

9. A device according to any one of claims 1 to 8, **characterized in that** each well (6) presents a hydrating calibrated open section defined by an outline of shape selected in such a manner that none of the points taken inside said section is situated at a linear distance from the closest outline of said section that is greater than 4 mm.

10. A device according to any one of claims 1 to 9, **characterized in that** each well (6) presents a hydrating calibrated open section defined by an outline of shape that is selected in such a manner that the smallest dimension of said section is greater than 1.5 mm.

11. A device according to any one of claims 1 to 10, **characterized in that** each well (6) presents a hydrating calibrated open section defined by an outline of shape selected in such a manner that none of the points taken between the outside of said section and the outline of the absorption face (3₁) of the hydrating support is situated at a linear distance from the closest outlines of said face or of said section that is greater than 3 mm.

12. A device according to any one of claims 1 to 11, **characterized in that** the lid (5) is provided with a plurality of hydrating supports (3), each of which is to be hydrated by a respective liquid sample, and **in that** the number and the volume of wells (6) facing each hydrating support are identical for all of the hydrating supports.

13. A device according to any one of claims 1 to 12, **characterized in that** the well(s) (6) including the liquid sample reception mini-channels define a calibrated volume adapted so that in the hydrating position of the lid (4) and of the container (5), the liquid sample is absorbed completely by the hydrating support (3).

14. A device according to any one of claims 1 to 13, **characterized in that** the well(s) (6) including the liquid sample reception microchannels (16) define a calibrated volume adapted so that in the hydrating position of the lid (4) and of the container (5), the hydrating support (3) is completely hydrated.

15. A device according to any one of claims 1 to 14, **characterized in that** at least one well (6) presents a profile that tapers going away from its top edge (8).

16. A device according to any one of claims 1 to 15, **characterized in that** the container (4) includes a plate (9) in which the well(s) and the microchannels are arranged, so as to be surrounded by respective peripheral rim(s) (12) that serve in the hydrating position as abutment surfaces for the hydrating supports.

17. A device according to any one of claims 1 to 16, **characterized in that** the container (4) includes a plate (9) from which a peripheral margin (14) surrounding the wells projects upwards defining internally at least one distribution bowl (13) for distributing the liquid sample(s).

18. A device according to claim 17, **characterized in that** the lid (5) possesses a peripheral groove (19) suitable for engaging on the peripheral margin (14) of the container (4) in order to provide engagement that is leaktight or that includes controlled ventilation.

19. A device according to any one of claims 1 to 18, **characterized in that** it includes a centering system (23) for relative centering between the lid (5) and the container (4) in order to position each hydrating support (3) in a position that is superposed relative to one or more wells (6) for receiving a respective liquid sample (2).

20. A device according to any one of claims 1 to 19, **characterized in that** it includes a locking system (24) for locking the lid (5) and the container (4) in a stable closed position corresponding to the hydrating position.

21. A device according to any one of claims 1 to 20, **characterized in that** the container (4) and/or the lid (5) are made of a material that is transparent or translucent.

22. A device according to any one of claims 1 to 21, **characterized in that** each well (6) is provided with a stain for the liquid sample in order to assist in filling said well.

23. The use of a device (1) according to any one of claims 1 to 22, in order to detect and/or identify and/or enumerate at least one microorganism in a liquid sample (2) that might contain it.

24. The use of a device (1) according to any one of claims 1 to 22, in order to determine the sensitivity and the resistance of at least one microorganism to at least one antibiotic.

25. A method of hydrating at least one hydrating support with a liquid sample (2) by using a device (1) in accordance with any one of claims 1 to 22 in order to detect and/or identify and/or enumerate at least one target microorganism in a liquid sample that might contain it, the method being **characterized in that** it comprises the following steps:
- completing distributing each liquid sample (2) in one or more wells (6);
- positioning the lid (5) and the container (4) in their hydrating position in which each hydrating support (3) is in contact with a liquid sample (2);
- maintaining the lid (5) and the container (4) in the hydrating position in order to perform an incubation step; and
- reading each hydrating support (3) in order to detect and/or identify and/or enumerate at least one target microorganism.

26. A method according to claim 25, **characterized in that** it consists in distributing each liquid sample (2) by pouring the liquid sample(s) into the container (4) and by agitating the container (4) in order to ensure that each liquid sample (2) completely fills the reception well(s) (6).

27. A method according to claim 25 or claim 26, **characterized in that** it consists in performing the step of reading each hydrating support (3) through the container (4) and/or the lid (5).

28. A method according to any one of claims 25 to 27, **characterized in that**, after the incubation step, it consists in replacing the container (4) with a plane bottom that is transparent or translucent through which the operation of reading each hydrating support (3) is performed.
